# FASCICULE DE BREVET EUROPEEN

(11) **EP 3 609 573 B1**
(45) Date de publication et mention de la délivrance du brevet: **19.03.2025**
(21) Numéro de dépôt: 18717620.1
(22) Date de dépôt: 13.04.2018
(51) Int. Cl.: A61N 5/06

(54) **DISPOSITIF D'IRRADIATION TRANSCUTANÉE POUR TRAITEMENT DES MALADIES NEURODÉGÉNÉRATIVES**
VORRICHTUNG ZUR TRANSKUTANEN BESTRAHLUNG ZUR BEHANDLUNG VON NEURODEGENERATIVEN KRANKHEITEN
TRANSCUTANEOUS IRRADIATION DEVICE FOR THE TREATMENT OF NEURODEGENERATIVE DISEASES

(30) Priorité: 14.04.2017 EP 17305450
(43) Date de publication de la demande: 19.02.2020
(73) Titulaire: REGENLIFE, 75008 Paris (FR)
(72) Inventeur: BLIVET, Guillaume, 34070 Montpellier (FR); MOREAU, Guillaume, 34670 Baillargues (FR)
(74) Mandataire: Regimbeau
(86) Numéro de dépôt international: PCT/EP2018/059596
(87) Numéro de publication internationale: WO 2018/189393

(56) Documents cités:
- EP-A1- 1 074 275
- US-A1- 2005 024 853
- US-A1- 2007 129 776
- US-A1- 2009 030 489
- US-A1- 2010 204 762

## Description

### DOMAINE DE L'INVENTION

L'invention s'inscrit dans le domaine du traitement par irradiation transcutanée.

L'invention porte plus particulièrement sur un dispositif permettant la mise en œuvre d'un traitement par irradiation transcutanée.

Le domaine d'application du dispositif de l'invention s'inscrit dans le domaine du traitement des maladies neurologiques.

### ART ANTÉRIEUR

L'irradiation transcutanée est une technique connue par laquelle un rayonnement d'ondes ou de particules est émis au contact de la peau et pénètre en profondeur.

Parmi ces techniques, on connait notamment la photobiomodulation et la thérapie laser de basse intensité (en anglais, *LLLT, Low Level Laser Therapy*) utilisant respectivement des diodes électroluminescentes (LEDs) et des diodes lasers, et permettant de réparer et régénérer des tissus endommagés. Cette technique consiste à positionner une sonde sur la peau d'un patient et à réaliser l'émission photonique pendant un temps donné au niveau de la zone tissulaire endommagée. La sonde, par exemple commercialisée par la société THOR, comporte une tête d'émission placée sur la peau, une poignée de maintien de la tête d'émission et des câbles d'alimentation reliés à une unité de contrôle. La tête d'émission est maintenue en place par le praticien pendant toute la séance.

Ces techniques d'irradiations transcutanées, notamment par photothérapie, s'appliquent également aux traitements neurologiques et/ou psychiatriques. On parle alors d'irradiation transcrânienne. Dans ce cas, une sonde d'émission lumineuse allant du visible à l'infrarouge, du type de celle précédemment décrite, est positionnée et maintenue à la surface de la tête du patient par le praticien. Il est possible d'agir par cette technique sur des troubles neurologiques de façon thérapeutique pour restaurer les facultés neurologiques, stopper la progression des troubles cognitifs, par exemple touchant les maladies neurodégénératives de type Alzheimer, ou maintenir une qualité de vie.

En outre, l'application de ces techniques aux traitements neurologiques nécessite un positionnement précis de la source d'irradiation dirigée vers une partie du cerveau impliquée dans le trouble à soigner. Il s'agit naturellement de viser uniquement la zone concernée, au risque d'affecter une zone voisine. Pour ce faire, un dispositif connu et commercialisé par la société NEUROTHERA prévoit un support de sonde formant un casque et comportant des anneaux répartis sur la tête du patient. Le casque est maintenu en position au moyen d'une sangle comportant une mentonnière qui s'attache de chaque côté à deux anneaux périphériques. Chaque anneau est un indicateur de position vers une zone cible du cerveau. La sonde est positionnée par le praticien au niveau d'un anneau et maintenue dans cette position pendant la durée du traitement.

Enfin, une autre technique utilisée dans le traitement d'affections neurologiques ou psychiatrique est la stimulation magnétique transcrânienne. Cette technique consiste à appliquer une ou une série d'impulsions magnétiques sur le cortex cérébral. Des effets ont été observé dans le traitement de la maladie d'Alzheimer (Koch et al., 2018. Neuroimage. 169:302-311), de certaines dégénérescences lobaires fronto-temporales (Antczak et al., 2018. Neuropsychiatr Dis Treat. 14:749-755) ou encore de la dépression (Pridmore & Pridmore, 2018. Aust J Gen Pract. 47(3):122-125).

Des dispositifs d'irradiation transcutanée sont connus par exemple de EP1074275 ou US2005/024853.

Ici, les Inventeurs ont développé une approche multi-cible et multimodale, combinant à la fois la photobiomodulation, la thérapie laser de basse intensité ainsi que la stimulation magnétique. De manière surprenante, la combinaison des cibles anatomiques traitées et de ces techniques offre un effet synergique dans le traitement de maladies neurodégénératives, comme Alzheimer, Parkinson ou Huntington.

### INCONVÉNIENTS DE L'ART ANTÉRIEUR

Les techniques d'irradiations telles que précédemment décrite ne permettent pas d'obtenir des résultats suffisamment satisfaisants sur l'évolution des troubles neurologiques.

En outre, le dispositif commercialisé par la société NEUROTHERA est insuffisant pour assurer un positionnement précis de l'émission lumineuse vers une zone cible. En effet, le maintien de la sonde par le praticien peut engendrer un changement d'angle de l'émission lumineuse même lorsque la sonde reste en position dans l'anneau. En outre, le praticien doit nécessairement maintenir la sonde pendant le traitement. Il n'est d'ailleurs pas possible, dans ce système, d'effectuer des traitements simultanés de plusieurs zones du cerveau au regard du fait que chaque sonde est maintenue en place par le praticien et qu'un dispositif de contrôle encombrant est en liaison avec la sonde. Pourtant, l'efficacité de certains traitements neurologiques peut être conditionnée par la possibilité de pouvoir réaliser des irradiations sur plusieurs zones en même temps pour stimuler de façon concomitante des parties ciblées du cerveau.

### OBJECTIFS DE L'INVENTION

L'invention vise en premier lieu un dispositif d'irradiation transcutanée présentant une efficacité substantielle sur les symptômes des maladies neurologiques et/ou psychiatriques telles que la maladie d'Alzheimer.

L'invention vise en outre un dispositif permettant de réaliser l'irradiation transcutanée sur des zones précises du corps. Notamment, l'invention vise un système assurant un positionnement adapté à la morphologie de la tête et de l'abdomen du patient de la ou des sources d'irradiations.

### EXPOSÉ DE L'INVENTION

À cet effet, le dispositif d'irradiation transcutanée de l'invention est défini dans les revendications attenantes et comprend une partie supérieure (1) apte à être positionnée sur la tête (3) d'un utilisateur, laquelle partie supérieure comprend au moins un module d'irradiation transcutanée (10) composé d'au moins une source d'irradiation (14a, 14b, 14c) ; et une partie inférieure (9) apte à être positionnée sur l'abdomen (16) de l'utilisateur, laquelle partie inférieure comprend au moins un module d'irradiation transcutanée (10) composé d'au moins une source d'irradiation (14a, 14b, 14c) et chaque module d'irradiation transcutanée (10) comprenant au moins une source laser de type pulsé (14a), une fréquence de modulation globale étant appliquée aux sources d'irradiation, la source laser de type pulsé étant en conséquence soumise à une double pulsation : une première pulsation, intrinsèque et une seconde pulsation, extrinsèque et résultant de l'application de la fréquence de modulation globale à la première pulsation.

Le dispositif de l'invention peut également comprendre les caractéristiques optionnelles suivantes considérées isolément ou selon toutes les combinaisons techniques possibles :
- la source laser de type pulsé (14a) génère un faisceau émettant dans le spectre infrarouge, ledit faisceau présentant une longueur d'onde comprise entre 700 et 1200 nanomètres et un train d'impulsion comprenant :
   ∘ une durée d'impulsion comprise entre 20 et 200 nanosecondes ;
   ∘ une fréquence de répétition du train d'impulsion comprise entre 1 et 25 kHz inclus ;
   ∘ une puissance impulsionnelle comprise entre 0,5 et 12 Watts inclus ; et
   ∘ une tension comprise entre 2 et 5 Volts inclus ;
- le faisceau présente une longueur d'onde comprise entre 800 et 900 nanomètres, de préférence d'environ 850 nanomètres ;
- la durée d'impulsion du faisceau généré par la source laser (14a) est comprise entre 75 et 150 nanosecondes, de préférence entre 90 et 110 nanosecondes ;
- la fréquence de répétition du faisceau généré par la source laser (14a) est comprise entre 10 et 15 kHz inclus, de préférence est d'environ 15 kHz ;
- la puissance impulsionnelle du faisceau généré par la source laser (14a) est comprise entre 1 et 7 Watts inclus ;
- chaque module d'irradiation transcutanée (10) comprend au moins une diode électroluminescente (14b, 14c) générant un faisceau émettant dans le spectre visible ou dans le spectre infrarouge ;
- chaque module d'irradiation transcutanée (10) comprend au moins :
   ∘ une source laser de type pulsé (14a) générant un faisceau émettant dans le spectre infrarouge ;
   ∘ une diode électroluminescente ou une source laser générant un faisceau émettant dans le spectre rouge (14b), et ;
   ∘ une diode électroluminescente générant un faisceau émettant dans le spectre infrarouge (14c) ;
- le faisceau émettant dans le spectre rouge généré par la au moins une diode électroluminescente ou source laser (14b) présente une longueur d'onde comprise entre 600 et 700 nanomètres, et en ce que le faisceau émettant dans le spectre infrarouge généré par la au moins une diode électroluminescente (14c) présente une longueur d'onde entre 700 et 1200 nanomètres ;
- le faisceau émettant dans le spectre rouge généré par la au moins une diode électroluminescente ou source laser (14b) présente une longueur d'onde comprise 620 et 650 nanomètres, de préférence d'environ 640 nanomètres ou d'environ 625 nm ;
- le faisceau émettant dans le spectre infrarouge généré par la au moins une diode électroluminescente (14c) présente une longueur d'onde d'environ 850 nanomètres ;
- chaque module d'irradiation transcutanée (10) comprend en outre une source générant un champ magnétique statique (18), de préférence un aimant ou un électro-aimant ;
- la source générant un champ magnétique statique (18) comprend une forme d'anneau destiné à être agencer perpendiculairement au plan duquel est générée l'irradiation transcutanée par la au moins une source d'irradiation (14a, 14b, 14c) ;
- le dispositif comprend des moyens d'application de l'irradiation par la au moins une source d'irradiation (14a, 14b, 14c) à des fréquences inférieures à 1000 Hertz (inclus), de préférence comprises entre 1 et 1000 Hertz (inclus) ;
- les moyens d'applications de l'irradiation comprennent un module de synchronisation des émissions du module d'irradiation transcutanée de la partie supérieure (1) avec les émissions du module d'irradiation transcutanée de la partie inférieure (9), lesdits faisceaux étant émis à une fréquence de modulation globale :
   ∘ d'environ 9 à 11 Hz, de préférence d'environ 10 Hz pour chacun des modules d'irradiation transcutanée (10) de la partie supérieure (1) et de la partie inférieure (9) ; ou
   ∘ d'environ 9 à 11 Hz, de préférence d'environ 10 Hz pour chacun des modules d'irradiation transcutanée (10) de la partie supérieure (1) et d'environ 900 à 1000 kHz, de préférence d'environ 1000 Hz pour chacun des modules d'irradiation transcutanée (10) de la partie inférieure (9) ;
- le dispositif comprend au moins deux modules d'irradiation transcutanée (10) disposés symétriquement sur la tête de l'utilisateur, et au moins deux modules d'irradiation transcutanée (10) disposés sur l'abdomen de l'utilisateur, de préférence disposés symétriquement ;
- au moins une source d'irradiation (14a, 14b, 14c) est maintenue au sein d'un moyen de maintien dont la forme est sensiblement cylindrique, ledit moyen de maintien étant destiné à être au contact d'un patient de sorte que le faisceau soit émis parallèlement à l'axe du cylindre ;
- le dispositif comprend au moins deux modules d'irradiation disposés symétriquement sur la tête de l'utilisateur, et au moins deux modules d'irradiation disposés sur l'abdomen de l'utilisateur, de préférence disposés symétriquement ;
- chaque module est fixé à un support de module qui comprend des moyens de positionnement dudit support au niveau de la zone à irradier, et au moins un anneau réalisé en un matériau élastique et/ou souple et apte à assurer la fixation par emprise élastique dudit module d'irradiation ;
- le support de module comprend une pluralité d'anneaux reliés entre eux par des éléments de jonction en formant un support de module d'irradiation transcrânienne apte à être positionné sur la tête d'un utilisateur ;
- les anneaux sont symétriquement disposés de part et d'autre d'un axe coïncidant avec l'axe médian de la tête lorsque le support est en place sur la tête de l'utilisateur, les anneaux comprennent des anneaux périphériques dont certains au moins ne sont pas reliés entre eux par des éléments de jonction ;
- les anneaux périphériques ne sont pas reliés entre eux par des éléments de jonction ;
- les anneaux comprennent des anneaux de seconde périphérie et quatre anneaux centraux, au moins certains anneaux de seconde périphérie ne sont pas reliés entre eux par des éléments de jonction, et les quatre anneaux centraux sont reliés entre eux par des éléments de jonction ;
- le support comprend une sangle à serrage contrôlé comprenant au moins une partie de liaison reliant les anneaux périphériques ;
- la sangle comprend une mentonnière se prolongeant de chaque côté du support jusqu'à deux éléments de liaisons qui sont destinés à être respectivement disposés de chaque côté d'une oreille de l'utilisateur et qui forment jonction entre ladite mentonnière et ladite partie de liaison ;
- la face interne de chaque anneau comprend une rainure d'aide à la fixation du module dédié ;
- les anneaux sont réalisés en un matériau souple et/ou élastique ;
- le dispositif de l'invention comprend un support tel que précédemment décrit, et le ou les modules d'irradiation sont aptes à être coaxialement fixé sur l'anneau du support.

L'invention porte également sur un système comprenant le dispositif de l'invention et une console de pilotage comprenant une interface de commande pour configurer les paramètres de chacune des au moins une source d'irradiation (14a, 14b, 14c) et une interface de communication pour délivrer des consignes numériques de pilotage au dit dispositif.

L'invention porte également sur le dispositif ou le système selon la présente invention, configuré pour la prévention ou le traitement des troubles neurologiques et/ou des maladies neurodégénératives, de préférence pour la prévention ou le traitement de la maladie d'Alzheimer, de la maladie de Parkinson et/ou de la maladie de Huntington.

La présente divulgation porte en outre sur l'application du dispositif tel que précédemment décrit pour le traitement des troubles neurologiques et/ou le traitement des maladies neurodégénératives, par exemple de type maladie d'Alzheimer, maladie de Parkinson ou maladie de Huntington, une telle application ne faisant néanmoins pas partie de l'objet revendiqué.

### PRÉSENTATION DES FIGURES

D'autres caractéristiques et avantages de l'invention ressortiront clairement de la description qui en est donnée ci-dessous, à titre indicatif et nullement limitatif, en référence aux figures annexées.
La **Figure 1** est une vue schématique de coté de la partie supérieure du dispositif de l'invention positionnée sur la tête de l'utilisateur et comprenant, dans cet exemple, quatre modules d'irradiation (dont deux seulement sont visibles sur cette figure).
La **Figure 2** est une vue schématique de face de la partie inférieure du dispositif de l'invention positionnée sur l'abdomen de l'utilisateur et comprenant dans cet exemple quatre modules d'irradiation.
La **Figure 3** est une vue en coupe selon la ligne III-III de la **Figure 1** d'un module d'irradiation du dispositif de l'invention selon une première variante, en position sur la peau de la tête ou de l'abdomen d'un utilisateur.
La **Figure 4** est une représentation de dessus de la carte électronique d'un module d'irradiation du dispositif de l'invention selon une seconde variante, illustrant la position et qualité des sources d'irradiation.
La **Figure 5** est une vue schématique de dessus du support de modules de la partie supérieure du dispositif de l'invention destinée à être positionnée sur la tête de l'utilisateur.
La **Figure 6** est une vue schématique de côté du support de modules de la partie supérieure du dispositif de l'invention destinée à être positionnée sur la tête de l'utilisateur.
La **Figure 7** est une vue schématique de dessus du support de modules de la partie supérieure du dispositif de l'invention en position sur la tête de l'utilisateur.
La **Figure 8** est une vue schématique de face du support de modules de la partie supérieure du dispositif de l'invention en position sur la tête de l'utilisateur.
La **Figure 9** est une vue schématique de dos du support de modules de la partie supérieure du dispositif de l'invention en position sur la tête de l'utilisateur.
La **Figure 10** est une vue schématique de côté du support de modules de la partie supérieure du dispositif de l'invention en position sur la tête de l'utilisateur.
La **Figure 11** est un schéma illustrant l'altération de la mémoire spatiale spontanée de souris auxquelles a été injecté le peptide amyloïde Aβ₂₅₋₃₅, pour des souris soumises à un traitement d'irradiation réalisé par différents dispositifs selon l'invention et pour des souris témoins non soumises à un traitement d'irradiation.
La **Figure 12** est un schéma illustrant l'altération de la mémoire spatiale à long terme selon un premier test de souris auxquelles a été injecté le peptide amyloïde Aβ₂₅₋₃₅, pour des souris soumises à un traitement d'irradiation réalisé par différents dispositifs selon l'invention et pour des souris témoins non soumises à un traitement d'irradiation.
La **Figure 13** est un schéma illustrant l'altération de la mémoire spatiale à long terme selon un second test de souris auxquelles a été injecté le peptide amyloïde Aβ₂₅₋₃₅, pour des souris soumises à un traitement d'irradiation réalisé par différents dispositifs selon l'invention et pour des souris témoins non soumises à un traitement d'irradiation.
La **Figure 14** est un schéma illustrant le taux de peroxydation lipidique dans l'hippocampe de souris auxquelles a été injecté le peptide amyloïde Aβ₂₅₋₃₅, pour des souris soumises à un traitement d'irradiation réalisé par différents dispositifs selon l'invention et pour des souris témoins non soumises à un traitement d'irradiation.
La **Figure 15** est un schéma illustrant le taux de GFAP (protéine acide fibrillaire gliale) mesuré par la méthode immuno-enzymatique ELISA dans l'hippocampe de souris auxquelles a été injecté le peptide amyloïde Aβ₂₅₋₃₅, pour des souris soumises à un traitement d'irradiation réalisé par différents dispositifs selon l'invention et pour des souris témoins non soumises à un traitement d'irradiation.
La **Figure 16** est un schéma illustrant le taux de TNFα (facteur de nécrose tumorale) mesuré par la méthode immuno-enzymatique ELISA dans l'hippocampe de souris auxquelles a été injecté le peptide amyloïde Aβ₂₅₋₃₅, pour des souris soumises à un traitement d'irradiation réalisé par différents dispositifs selon l'invention et pour des souris témoins non soumises à un traitement d'irradiation.
La **Figure 17** est, comme pour la **Figure 11****,** un schéma illustrant l'altération de la mémoire spatiale spontanée de souris auxquelles a été injecté le peptide amyloïde Aβ₂₅₋₃₅, d'une part pour des souris témoins non soumises à un traitement d'irradiation et d'autre part pour des souris soumises à un traitement d'irradiation par :
   - un dispositif selon l'invention comprenant des modules d'irradiation pourvus de diodes électroluminescentes, de laser pulsé et d'un aimant, en appliquant des temps de traitement de variant de 2,5 à 10 minutes ; et
   - un dispositif en dehors du champ de l'invention comprenant les mêmes modules d'irradiation uniquement appliqués sur la tête de l'utilisateur.

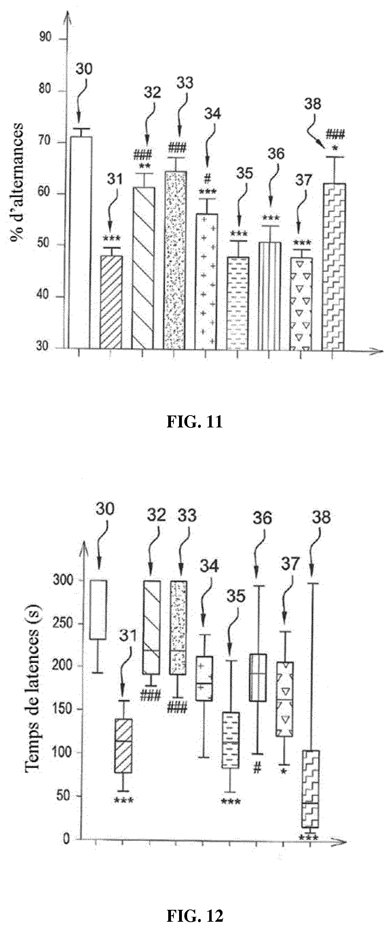

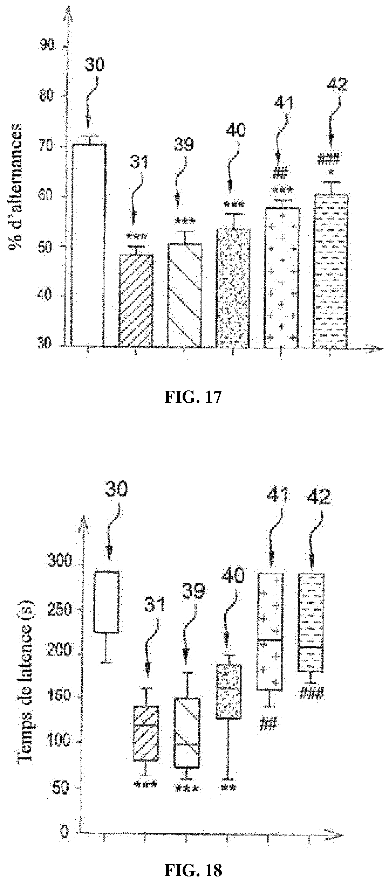

La **Figure 18** est, comme pour la **Figure 12****,** un schéma illustrant l'altération de la mémoire spatiale à long terme selon un premier test de souris auxquelles a été injecté le peptide amyloïde Aβ₂₅₋₃₅, d'une part pour des souris témoins non soumises à un traitement d'irradiation et d'autre part pour des souris soumises à un traitement d'irradiation selon les mêmes dispositifs que ceux évoqués en référence à la **Figure 17****.**

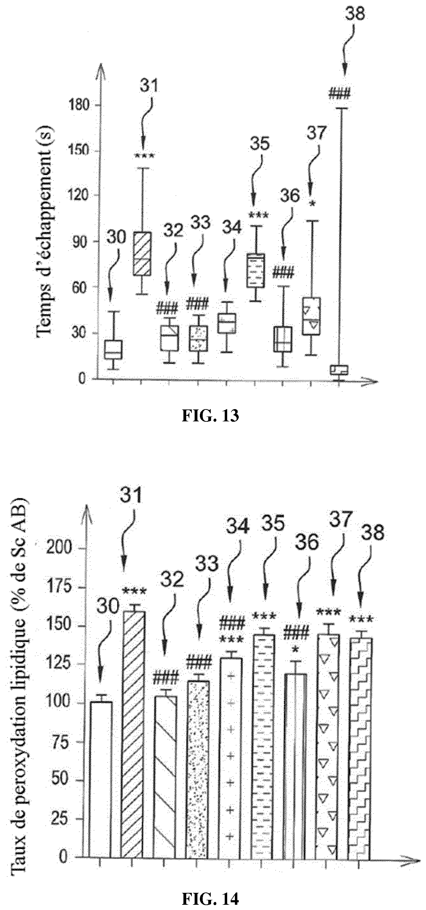

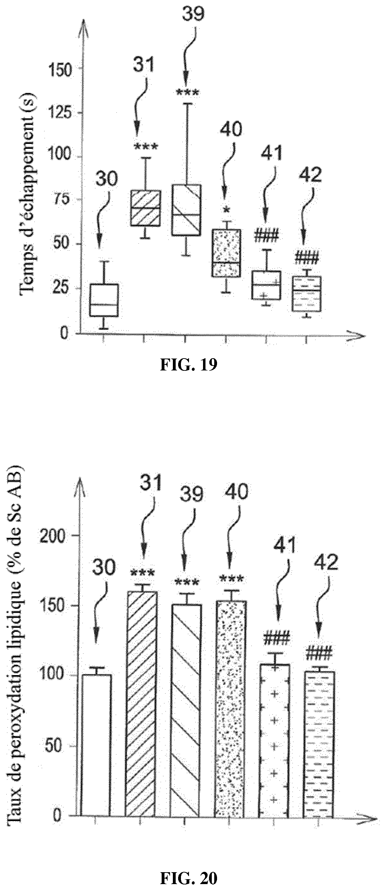

La **Figure 19** est, comme pour la **Figure 13****,** un schéma illustrant l'altération de la mémoire spatiale à long terme selon un second test de souris auxquelles a été injecté le peptide amyloïde Aβ₂₅₋₃₅, d'une part pour des souris témoins non soumises à un traitement d'irradiation et d'autre part pour des souris soumises à un traitement d'irradiation selon les mêmes dispositifs que ceux évoqués en référence à la **Figure 17****.**
La **Figure 20** est, comme pour la **Figure 14****,** un schéma illustrant le taux de peroxydation lipidique dans l'hippocampe de souris auxquelles a été injecté le peptide amyloïde Aβ₂₅₋₃₅, d'une part pour des souris témoins non soumises à un traitement d'irradiation et d'autre part pour des souris soumises à un traitement d'irradiation selon les mêmes dispositifs que ceux évoqués en référence à la **Figure 17****.**

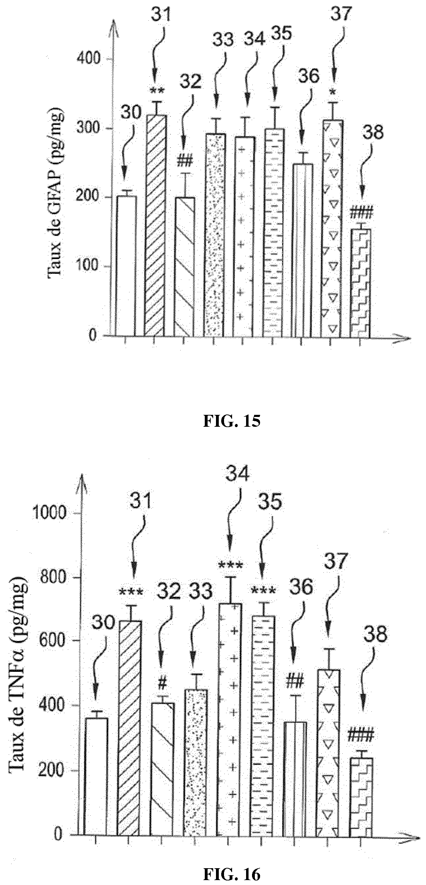

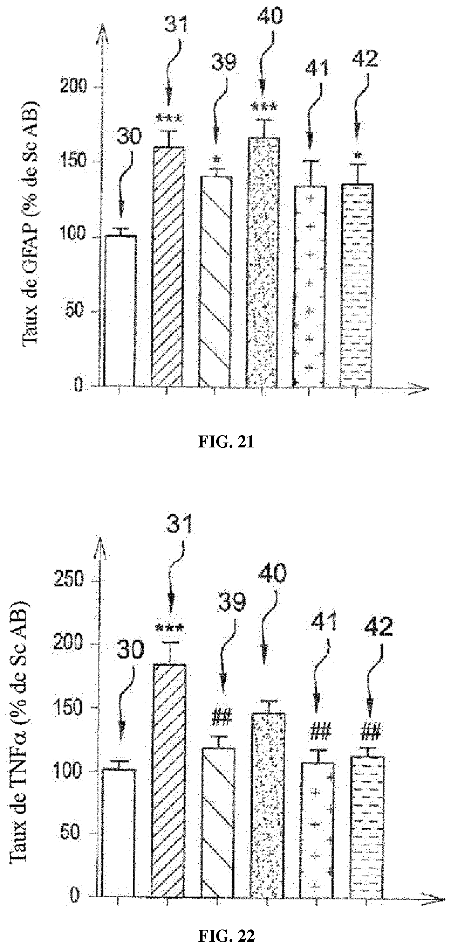

La **Figure 21** est, comme pour la **Figure 15****,** un schéma illustrant le taux de GFAP (protéine acide fibrillaire gliale) mesuré par la méthode immuno-enzymatique ELISA dans l'hippocampe de souris auxquelles a été injecté le peptide amyloïde Aβ₂₅₋₃₅, d'une part pour des souris témoins non soumises à un traitement d'irradiation et d'autre part pour des souris soumises à un traitement d'irradiation selon les mêmes dispositifs que ceux évoqués en référence à la **Figure 17****.**
La **Figure 22** est, comme pour la **Figure 16****,** un schéma illustrant le taux de TNFα (facteur de nécrose tumorale) mesuré par la méthode immuno-enzymatique ELISA dans l'hippocampe de souris auxquelles a été injecté le peptide amyloïde Aβ₂₅₋₃₅, d'une part pour des souris témoins non soumises à un traitement d'irradiation et d'autre part pour des souris soumises à un traitement d'irradiation selon les mêmes dispositifs que ceux évoqués en référence à la **Figure 17****.**

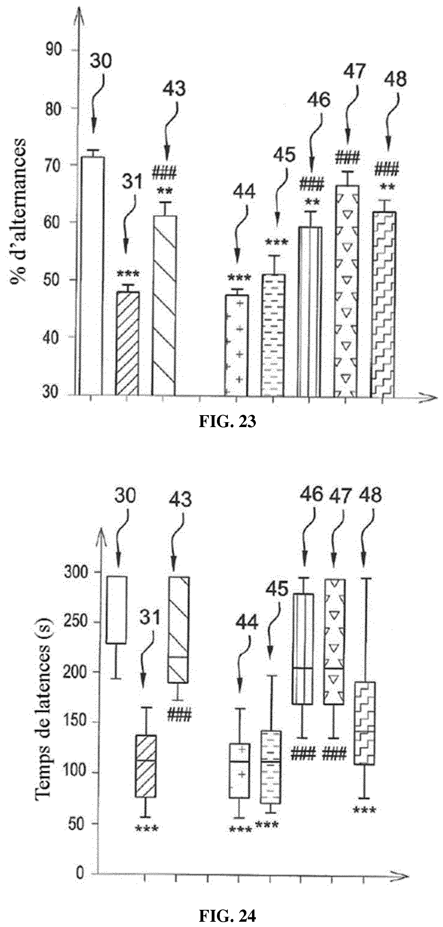

La **Figure 23** est, comme pour les **Figures 11** et **17****,** un schéma illustrant l'altération de la mémoire spatiale spontanée de souris auxquelles a été injecté le peptide amyloïde Aβ₂₅₋₃₅, d'une part pour des souris témoins non soumises à un traitement d'irradiation et d'autre part pour des souris soumises à un traitement d'irradiation par :
   - un dispositif selon l'invention comprenant des modules d'irradiation pourvus de diodes électroluminescentes, de laser pulsé et d'un aimant, en appliquant différentes fréquences d'irradiation comprises entre 0 et 1000 Hertz ; et
   - des dispositifs en dehors du champ de l'invention comprenant des modules d'irradiation uniquement appliqués sur la tête ou sur l'abdomen de l'utilisateur.
La **Figure 24** est, comme pour les **Figures 12** et **18****,** un schéma illustrant l'altération de la mémoire spatiale à long terme selon un premier test de souris auxquelles a été injecté le peptide amyloïde Aβ₂₅₋₃₅, d'une part pour des souris témoins non soumises à un traitement d'irradiation et d'autre part pour des souris soumises à un traitement d'irradiation selon les mêmes dispositifs que ceux évoqués en référence à la **Figure 23****.**

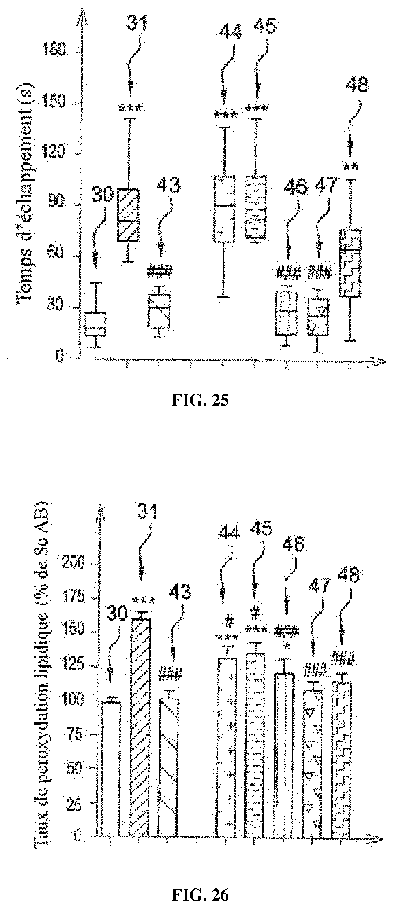

La **Figure 25** est, comme pour les **Figures 13** et **19****,** un schéma illustrant l'altération de la mémoire spatiale à long terme selon un second test de souris auxquelles a été injecté le peptide amyloïde Aβ₂₅₋₃₅, d'une part pour des souris témoins non soumises à un traitement d'irradiation et d'autre part pour des souris soumises à un traitement d'irradiation selon les mêmes dispositifs que ceux évoqués en référence à la **Figure 23****.**
La **Figure 26** est, comme pour les **Figures 14** et **20****,** un schéma illustrant le taux de peroxydation lipidique dans l'hippocampe de souris auxquelles a été injecté le peptide amyloïde Aβ₂₅₋₃₅, d'une part pour des souris témoins non soumises à un traitement d'irradiation et d'autre part pour des souris soumises à un traitement d'irradiation selon les mêmes dispositifs que ceux évoqués en référence à la **Figure 23****.**

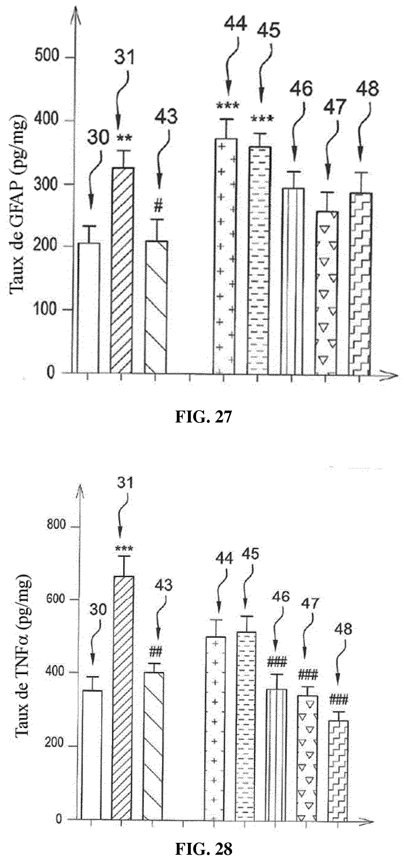

La **Figure 27** est, comme pour les **Figures 15** et **21****,** un schéma illustrant le taux de GFAP (protéine acide fibrillaire gliale) mesuré par la méthode immuno-enzymatique ELISA dans l'hippocampe de souris auxquelles a été injecté le peptide amyloïde Aβ₂₅₋₃₅, d'une part pour des souris témoins non soumises à un traitement d'irradiation et d'autre part pour des souris soumises à un traitement d'irradiation selon les mêmes dispositifs que ceux évoqués en référence à la **Figure 23****.**
La **Figure 28** est, comme pour les **Figures 16** et **22****,** un schéma illustrant le taux de TNFα (facteur de nécrose tumorale) mesuré par la méthode immuno-enzymatique ELISA dans l'hippocampe de souris auxquelles a été injecté le peptide amyloïde Aβ₂₅₋₃₅, d'une part pour des souris témoins non soumises à un traitement d'irradiation et d'autre part pour des souris soumises à un traitement d'irradiation selon les mêmes dispositifs que ceux évoqués en référence à la **Figure 23****.**

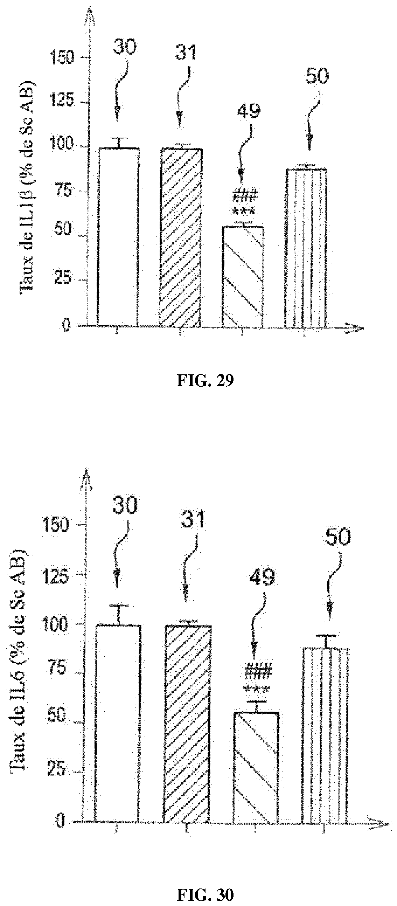

La **Figure 29** est un schéma illustrant le taux de IL1β (Interleukin 1 beta) dans le cortex frontal de souris auxquelles a été injecté le peptide amyloïde Aβ₂₅₋₃₅, d'une part pour des souris témoins non soumises à un traitement d'irradiation et d'autre part pour des souris soumises à un traitement d'irradiation par un dispositif selon l'invention comprenant des modules d'irradiation pourvus de diodes électroluminescentes, de laser pulsé et d'un aimant, et à un traitement d'irradiation par un dispositif selon l'invention identique mais dépourvu d'un aimant.
La **Figure 30** est un schéma illustrant le taux de IL6 (l'interleukine 6) dans le cortex frontal de souris auxquelles a été injecté le peptide amyloïde Aβ₂₅₋₃₅, d'une part pour des souris témoins non soumises à un traitement d'irradiation et d'autre part pour des souris soumises à un traitement d'irradiation selon les mêmes dispositifs que ceux évoqués en référence à la **Figure 29****.**

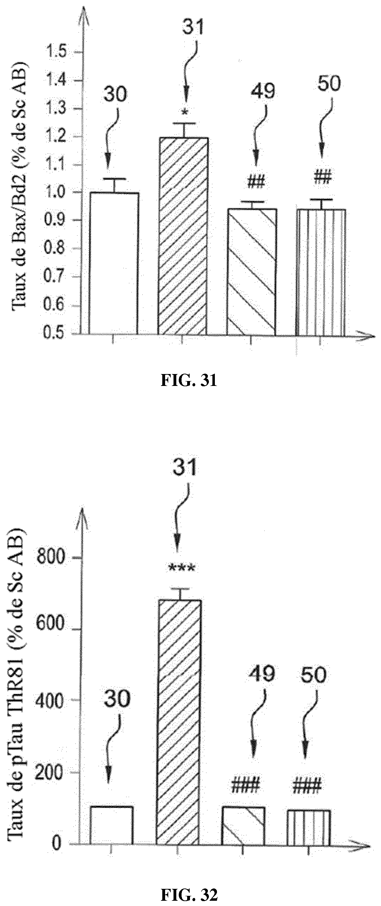

La **Figure 31** est un schéma illustrant le taux de protéine Bax/Bcl2 dans le cortex frontal de souris auxquelles a été injecté le peptide amyloïde Aβ₂₅₋₃₅, d'une part pour des souris témoins non soumises à un traitement d'irradiation et d'autre part pour des souris soumises à un traitement d'irradiation selon les mêmes dispositifs que ceux évoqués en référence à la **Figure 29****.**
La **Figure 32** est un schéma illustrant le taux de protéine Tau phosphorylée sur Thr81 (pTau Thr81) dans le cortex frontal de souris auxquelles a été injecté le peptide amyloïde Aβ₂₅₋₃₅, d'une part pour des souris témoins non soumises à un traitement d'irradiation et d'autre part pour des souris soumises à un traitement d'irradiation selon les mêmes dispositifs que ceux évoqués en référence à la **Figure 29****.**

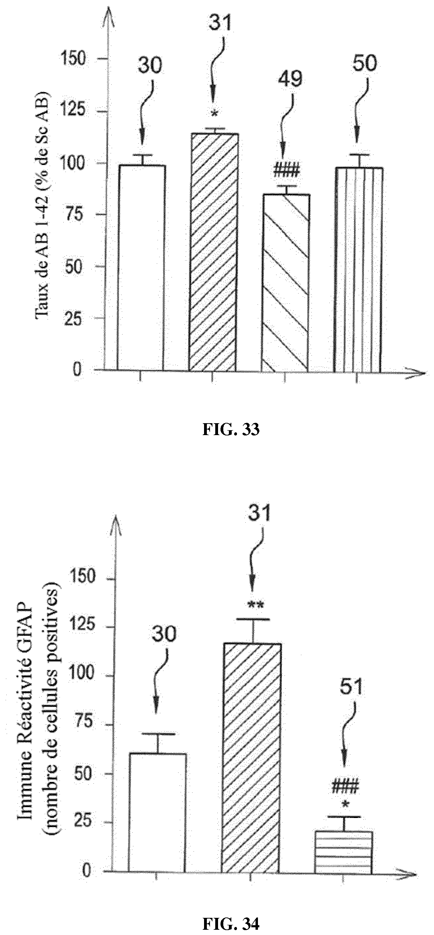

La **Figure 33** est un schéma illustrant le taux de Aβ₁₋₄₂ (protéine amyloïde-β (1-42)) dans le cortex frontal de souris auxquelles a été injecté le peptide amyloïde Aβ₂₅₋₃₅, d'une part pour des souris témoins non soumises à un traitement d'irradiation et d'autre part pour des souris soumises à un traitement d'irradiation selon les mêmes dispositifs que ceux évoqués en référence à la **Figure 29****.**
La **Figure 34** est un schéma illustrant l'activation des astrocytes observées dans la région CA1 de l'hippocampe dans une série de coupes histologiques de cerveau de souris injectées avec les oligomères Aβ₂₅₋₃₅, d'une part d'une part pour des souris témoins non soumises à un traitement d'irradiation et d'autre part pour des souris soumises à un traitement d'irradiation par un dispositif selon l'invention comprenant des modules d'irradiation pourvus de diodes électroluminescentes, de laser pulsé et d'un aimant.

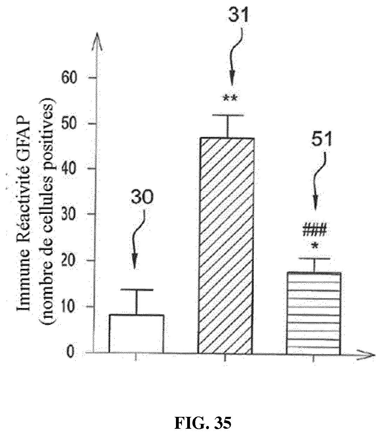

La **Figure 35** est un schéma illustrant le nombre de cellules microgliales activées observées dans la dans une série de coupes de cerveau dans la région CA1 de l'hippocampe dans une série de coupes histologiques de cerveau de souris injectées avec les oligomères Aβ₂₅₋₃₅, d'une part pour des souris témoins non soumises à un traitement d'irradiation et d'autre part pour des souris soumises à un traitement d'irradiation par le dispositif évoquée en référence à la **Figure 34****.**

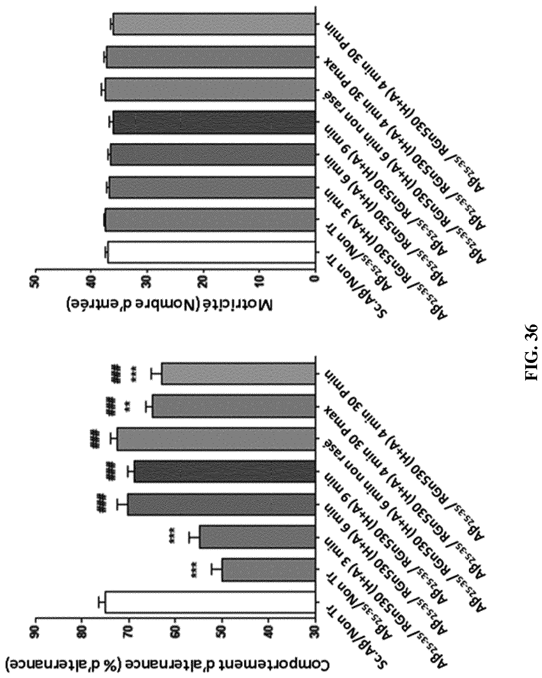

La **Figure 36** est un schéma illustrant la mémoire spatiale à court terme des souris (Y-*Maze test*). Le comportement d'alternance des souris dans le tunnel est représenté sur le schéma de gauche et la motricité des souris est représentée sur le schéma de droite. Ce test a été réalisé sur différents groupes de souris (n= 12 souris/groupes) auxquelles a été injecté le peptide amyloïde Aβ₂₅₋₃₅ ou le peptide contrôle (Sc.Aβ), d'une part pour des souris témoins non soumises à un traitement d'irradiation transcutanée et d'autre part pour des souris soumises à un traitement d'irradiation transcutanée à l'aide du dispositif « RGn530 ». L'analyse statistique a été réalisée à l'aide du test t de Dunn avec *p<0.05, **p<0.01, ***p<0.0001 (analyse par rapport au groupe peptide contrôle (Sc.Aβ₂₅₋₃₅) / Non traité) et #p<0.05, ##p<0.01, ### p<0.0001 (analyse par rapport au groupe peptide amyloïde Aβ₂₅₋₃₅) / non traité).

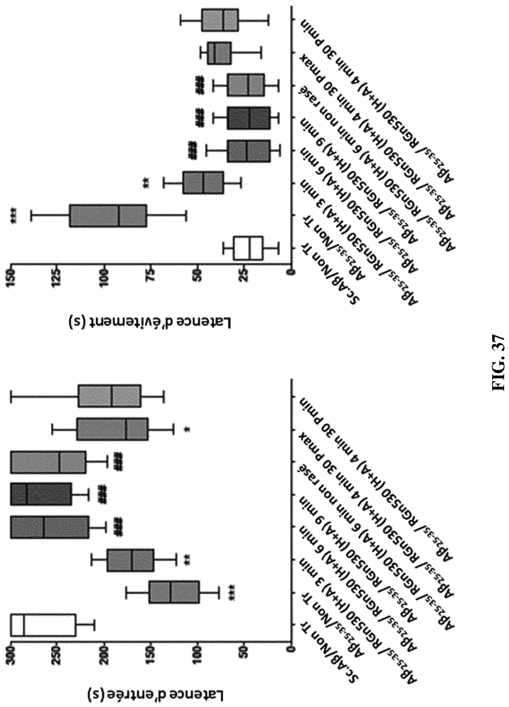

La **Figure 37** est un schéma illustrant la mémoire contextuelle à long terme des souris (test d'évitement passif). Le temps de latence d'entrée des souris dans le tunnel est représenté sur le schéma de gauche et le temps de latence d'évitement des souris est représenté sur le schéma de droite. Ce test a été réalisé sur différents groupes de souris (n= 12 souris/groupes) auxquelles a été injecté le peptide amyloïde Aβ₂₅₋₃₅ ou le peptide contrôle (Sc.Aβ), d'une part pour des souris témoins non soumises à un traitement d'irradiation et d'autre part pour des souris soumises à un traitement d'irradiation transcutanée à l'aide du dispositif « RGn530 ». L'analyse statistique a été réalisée à l'aide du test t de Dunn avec *p<0.05, **p<0.01, ***p<0.0001 (analyse par rapport au groupe peptide contrôle (Sc.Aβ₂₅₋₃₅) / véhicule (eau distillée)) et #p<0.05, ##p<0.01, ### p<0.0001 (analyse par rapport au groupe peptide amyloïde Aβ₂₅₋₃₅) / véhicule (eau distillée)).

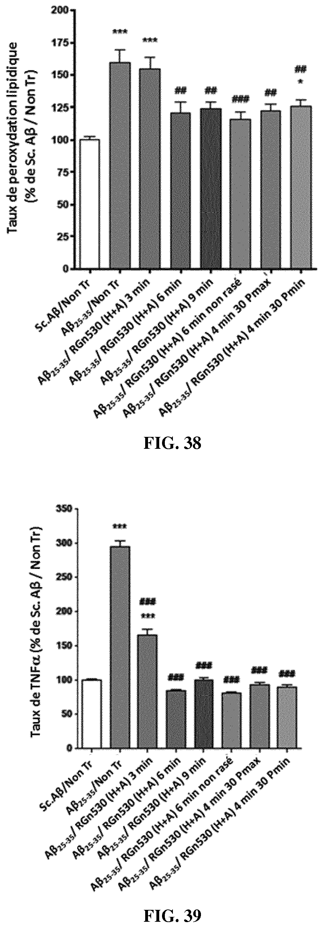

La **Figure 38** est un schéma illustrant le taux de peroxydation lipidique dans l'hippocampe de souris (n=12 souris par groupes) auxquelles a été injecté le peptide amyloïde Aβ₂₅₋₃₅ ou le peptide contrôle (Sc.Aβ), d'une part pour des souris témoins non soumises à un traitement d'irradiation et d'autre part pour des souris soumises à un traitement d'irradiation transcutanée à l'aide du dispositif « RGn530 ». L'analyse statistique a été réalisée à l'aide du test t de Dunn avec *p<0.05, **p<0.01, ***p<0.0001 (analyse par rapport au groupe peptide contrôle (Sc.Aβ₂₅₋₃₅) / véhicule (eau distillée)) et #p<0.05, ##p<0.01, ### p<0.0001 (analyse par rapport au groupe peptide amyloïde Aβ₂₅₋₃₅) / véhicule (eau distillée)).
La **Figure 39** est un schéma illustrant le taux de TNFα (Facteur de nécrose tumorale) mesuré par la méthode immuno-enzymatique ELISA dans l'hippocampe de souris (n=12 souris par groupes) auxquelles a été injecté le peptide amyloïde Aβ₂₅₋₃₅ ou le peptide contrôle, d'une part pour des souris témoins non soumises à un traitement d'irradiation et d'autre part pour des souris soumises à un traitement d'irradiation transcutanée à l'aide du dispositif « RGn530 ». L'analyse statistique a été réalisée à l'aide du test t de Dunn avec *p<0.05, **p<0.01, ***p<0.0001 (analyse par rapport au groupe peptide contrôle (Sc.Aβ₂₅₋₃₅) / véhicule (eau distillée)) et #p<0.05, ##p<0.01, ### p<0.0001 (analyse par rapport au groupe peptide amyloïde Aβ₂₅₋₃₅) / véhicule (eau distillée)).

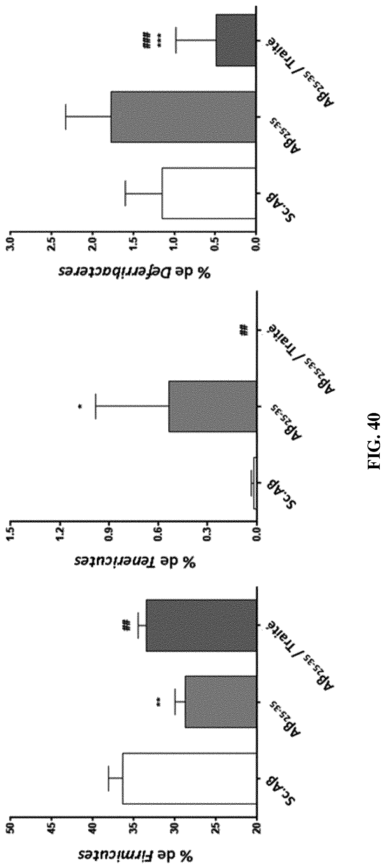

La **Figure 40** est un schéma illustrant l'abondance de trois phylum bactériens (*Firmicutes, Tenericutes* et *Deferribacteres*) analysés par 16S rDNA.dans le cœcum de souris (n=12 souris par groupes) auxquelles le peptide contrôle (Sc.Aβ₂₅₋₃₅) ou le peptide amyloïde Aβ₂₅₋₃₅ a été injecté sans traitement d'irradiation, et des souris auxquelles le peptide amyloïde A β₂₅₋₃₅ a été injecté et traitées par irradiation transcutanée à l'aide du dispositif « RGn530 » (6 minutes, souris rasé). L'analyse statistique a été réalisée à l'aide du test t de Mann-Whitney avec *p<0.05, **p<0.01, ***p<0.0001 (groupe peptide amyloïde Aβ₂₅₋₃₅ non traité par rapport au groupe contrôle (Sc.Aβ₂₅₋₃₅)) et #p<0.05, ##p<0.01, ### p<0.0001 (groupe peptide amyloïde Aβ₂₅₋₃₅ traité par rapport au groupe contrôle (Sc.AB₂₅₋₃₅)).

### DESCRIPTION DÉTAILLÉE DE L'INVENTION

### Le dispositif d'irradiation

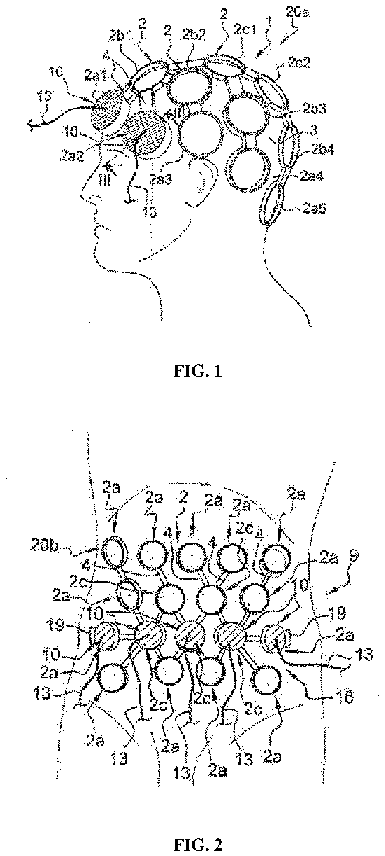

En référence aux **Figures 1, 2** et **3****,** le dispositif d'irradiation transcutanée de l'invention comprend deux parties. Une première partie supérieure 1 comprend un ou une pluralité de modules d'irradiation transcutanée 10 destinés à être positionnés sur la tête 3 de l'utilisateur ; et une seconde partie inférieure 9 comprend également un ou une pluralité de modules d'irradiation transcutanée 10 destinés à être positionnés sur l'abdomen 16 de l'utilisateur.

On entend par « positionné » au sens de la présente invention, le fait que les modules sont apposés sur la peau de l'utilisateur. Pour la partie supérieure du dispositif, les modules sont apposés sur la tête de l'utilisateur, et pour la partie inférieure du dispositif, les modules sont apposés sur l'abdomen de l'utilisateur.

Chaque module d'irradiation transcutanée 10 comprend un ou une pluralité de moyens d'irradiation 14 qui peuvent prendre la forme de sources laser de type pulsé 14a (de préférence, diode laser ou toute autre source émettrice de rayonnement laser connue de l'homme du métier) générant un faisceau émettant dans le spectre infrarouge, de diodes électroluminescentes (LED) ou de sources laser générant un faisceau émettant dans le spectre visible 14b et/ou de diodes électroluminescentes (LED) générant un faisceau émettant dans le spectre infrarouge 14c ; ainsi qu'éventuellement des moyens pour générer un champ magnétique statique, de préférence circulaire, comme par exemple un aimant 18 positionné au sein de chaque module d'irradiation transcutanée 10.

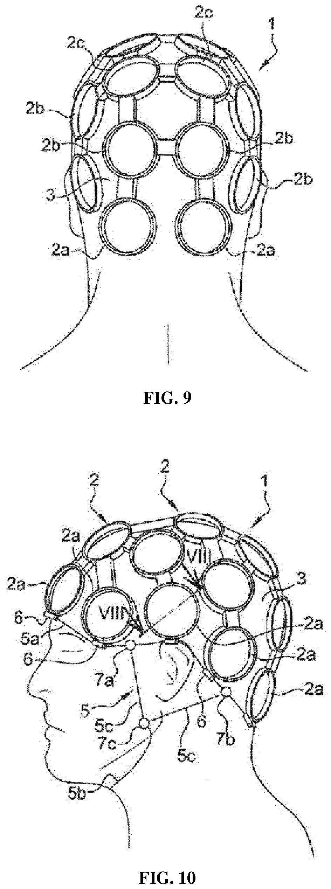

Comme représenté sur la **Figure 1** et pour ce mode de réalisation, la partie supérieure du dispositif 1 comprend quatre modules d'irradiation transcutanée 10 (dont deux seulement sont visibles sur cette figure) symétriquement disposés vers l'avant de la tête 3 pour atteindre le lobe frontal. Il peut être prévu moins de modules d'irradiation transcutanée 10 (au moins un selon l'invention) situés au niveau de n'importe quelle zone du cerveau, ou à l'inverse, plus de modules d'irradiation transcutanée 10 positionnés pour atteindre le lobe frontal, le lobe pariétal, le cervelet et/ou le lobe occipital. De préférence, le nombre de modules d'irradiation transcutanée 10 est supérieur à huit, dix, douze, quatorze, seize, dix-huit ou vingt, et peut être au plus de vingt-deux selon le mode de réalisation des **Figures 1** et **5** à **10.** De préférence, les modules d'irradiation transcutanée 10 se présentent sous un nombre pair et sont répartis sur la surface de la tête 3 de l'utilisateur de façon symétrique.

Comme représenté sur la **Figure 2****,** la partie inférieure du dispositif 9 comprend cinq modules d'irradiation transcutanée 10 positionnés symétriquement et alignés sensiblement horizontalement sur l'abdomen 16 de l'utilisateur. La zone de l'abdomen visée par le dispositif de la présente invention s'étend verticalement de l'aine à l'estomac et horizontalement sur la largeur de l'abdomen. Ainsi, comme pour la partie supérieure 1, il peut être prévu moins de modules d'irradiation transcutanée 10 (au moins un selon l'invention) situé au niveau de n'importe quelle zone de l'abdomen 16, ou à l'inverse, une pluralité de modules d'irradiation transcutanée 10 couvrant tout ou partie de l'abdomen 16 tel que précédemment défini. De préférence, le nombre de modules d'irradiation transcutanée 10 est supérieur à huit, dix, douze, quatorze ou seize, et peut être de dix-huit selon le mode de réalisation des **Figures 1** et **5** à **10.** Il est également possible de prévoir un nombre supérieur de modules d'irradiation transcutanée 10 (par exemple, un nombre de modules d'irradiation transcutanée 10 supérieur à dix-huit, vingt, vingt-deux ou plus) en adaptant la partie inférieure du dispositif 9 à un nombre de modules d'irradiation transcutanée 10 plus important. De préférence, les modules d'irradiation transcutanée 10 se présentent sous un nombre pair et sont répartis à la surface de l'abdomen 16 de l'utilisateur de façon symétrique.

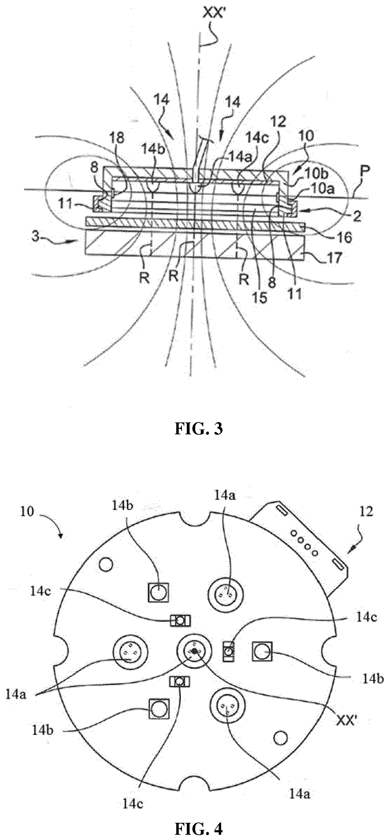

La **Figure 3** illustre une section d'un module d'irradiation transcutanée 10 positionné sur la tête 3 de l'utilisateur, mais s'applique également à un module d'irradiation transcutanée 10 positionné sur l'abdomen 16. Chaque module d'irradiation transcutanée 10 présente une forme générale cylindrique permettant d'assurer le maintien coaxial du module d'irradiation transcutanée 10 dans un anneau 2 formant partie d'un support de modules qui sera décrit en détail plus loin. Cette configuration coaxiale est importante car elle permet d'assurer le positionnement et le maintien fixe du module d'irradiation transcutanée 10 dans l'axe de l'anneau dédié 2, ce qui ne peut pas être le cas lorsque le module se présente, comme dans l'art antérieur, sous forme d'une sonde munie d'une poignée et de câbles reliés à dispositif de contrôle et d'alimentation, l'anneau formant alors dans ce cas uniquement la fonction d'indicateur de position.

L'anneau 2 est réalisé dans un matériau souple et/ou élastique et présente une rainure interne 8 dans laquelle vient se loger une nervure correspondante circulaire 11 ménagée sur la surface externe du module d'irradiation transcutanée 10 pour assurer le positionnement fixe et précis du module d'irradiation transcutanée 10 dans l'axe de l'anneau 2. La structure de l'anneau 2 ainsi que ses moyens de couplage avec le module d'irradiation transcutanée 10 peuvent être différents de ceux illustrés sur la **Figure 3** sans sortir du cadre de l'invention.

Dans un mode de réalisation, le module d'irradiation transcutanée 10 comprend une carte électronique 12 alimentée électriquement via un câble électrique 13 qui s'étend en dehors du module d'irradiation transcutanée 10. Ce câble électrique 13 est également représenté pour chaque module d'irradiation transcutanée 10 sur les **Figures 1** et **2****.** Dans un mode de réalisation alternatif, les anneaux 2 sont reliés entre eux par un câble d'alimentation unique 13 qui débouche au niveau de chaque anneau 2 par des moyens connus de l'homme du métier afin d'alimenter automatiquement chaque module d'irradiation transcutanée 10 positionné sur un anneau correspondant 2. Le câble électrique 13 renferme également des fils de données (par exemple de type bus CAN), un fil d'alimentation de la ou des sources d'irradiation et un ou plusieurs fils de masse.

Selon les deux modes de réalisation représentés sur les **Figures 3** et **4****,** chaque module d'irradiation transcutanée 10 du dispositif de l'invention comprend des sources d'irradiation consistant en au moins un laser pulsé 14a générant un faisceau émettant dans le spectre infrarouge, au moins une diode électroluminescente (LED) 14b générant un faisceau émettant dans le spectre rouge et au moins une diode électroluminescente (LED) 14c générant un faisceau émettant dans le spectre infrarouge. On peut alternativement utiliser une ou plusieurs sources laser 14b générant un faisceau émettant dans le spectre rouge en lieu et place ou en combinaison avec une ou plusieurs diodes électroluminescentes 14b générant un faisceau émettant dans le spectre rouge.

Pour le mode de réalisation représenté sur la **Figure 3****,** le module d'irradiation transcutanée 10 comprend un laser pulsé 14a qui est disposé dans l'axe principal XX' du module d'irradiation transcutanée 10, et deux LEDs générant un faisceau émettant respectivement dans le spectre rouge et infrarouge 14b, 14c qui sont situées symétriquement de part et d'autre du laser pulsé 14a.

Dans le second mode de réalisation préférentiel représenté sur la **Figure 4****,** le module d'irradiation transcutanée 10 comprend quatre lasers pulsés 14a, trois diodes électroluminescentes générant un faisceau émettant dans le spectre rouge 14b et trois diodes électroluminescentes générant un faisceau émettant dans le spectre infrarouge 14c.

Les sources d'irradiation 14 sont ainsi réparties en trois groupes, à savoir plus précisément :
- au moins une, préférentiellement deux, encore plus préférentiellement trois ou plus diodes électroluminescentes (LED) générant un faisceau émettant dans le spectre infrarouge 14c, ledit faisceau présentant une longueur d'onde comprise entre 700 et 1200 nanomètres, de préférence entre 800 et 900 nanomètres, de préférence d'environ 850 nanomètres. Les LEDs 14c sont circulairement et régulièrement réparties autour et à proximité de l'axe principal XX' du module ;
- au moins une, préférentiellement deux, encore plus préférentiellement trois ou plus diodes électroluminescente (LED) générant un faisceau émettant dans le spectre rouge 14b, ledit faisceau présentant une longueur d'onde comprise entre 600 et 700 nanomètres, de préférence d'environ 640 nanomètres ou de préférence d'environ 625 nm. Les LEDs 14b sont circulairement et régulièrement réparties autour et à plus grand distance que les LEDs 14c de l'axe principal XX' ; et
- au moins une, préférentiellement deux, encore plus préférentiellement trois, encore plus préférentiellement quatre ou plus sources laser (de préférence, diodes laser) de type pulsé 14a générant un faisceau émettant dans le spectre infrarouge, ledit faisceau présentant une longueur d'onde comprise entre 700 et 1200 nanomètres, de préférence entre 800 et 900 nanomètres, de préférence d'environ 850 nanomètres. Dans un mode de réalisation, chacune de ces sources laser 14a présente un train d'impulsion comprenant :
   ∘ une durée d'impulsion comprise entre 20 et 200 nanosecondes ;
   ∘ une fréquence de répétition du train d'impulsion comprise entre 1 et 25 kHz inclus, de préférence entre 10 et 15 kHz inclus, de préférence 15 kHz ou de préférence 10 kHz ;
   ∘ une puissance impulsionnelle comprise entre 0,5 et 12 Watts, de préférence entre 1 et 7 Watts inclus ; et
   ∘ une tension comprise entre 2 et 5 Volts inclus, de préférence 4,24 Volts ± 10% ou de préférence 2.30 Volt ± 10%.

Dans un mode de réalisation, une première source laser 14a est centrée sur l'axe XX' et les trois autres sources laser sont circulairement et régulièrement réparties autour et à plus grand distance que les LEDs 14c de l'axe principal XX', sur la même circonférence que les LEDs 14b. Dans un mode de réalisation alternatif, trois sources lasers 14a sont circulairement et régulièrement réparties autour de l'axe XX'. Dans un mode de réalisation alternatif, moins de trois sources laser 14a, par exemple, une unique source laser 14a est centrée sur l'axe XX' ou décentrée de l'axe XX'.

En restant dans le cadre de l'invention, les sources laser et les LEDs peuvent être disposées dans l'axe principal XX' ou désaxées, l'essentiel résidant dans le fait que l'illumination s'effectue dans la direction de la surface (tête ou abdomen) à irradier.

Dans un mode de réalisation préférentiel, le module d'irradiation transcutanée 10 comprend également des moyens non représentés mais connus de l'homme du métier assurant un mode pulsé, ou autrement dit, une « fréquence de modulation globale » appliquée aux diodes électroluminescentes 14b, 14c et aux sources laser 14a. Dans un mode de réalisation, la fréquence de modulation globale est comprise entre 0 et 4000 Hz, de préférence comprise entre 1 et 1000 Hz, de préférence comprise entre 1 et 100 Hz, de préférence d'environ 10 Hz.

Dans ce dernier mode préférentiel, une fréquence de modulation globale identique est appliquée aux sources d'irradiation 14a, 14b, 14c des modules d'irradiation transcutanée 10 appliqués sur la tête 3 de l'utilisateur et aux sources d'irradiation 14a, 14b, 14c des modules d'irradiation transcutanée 10 appliqués sur l'abdomen 16 de l'utilisateur. Ainsi, dans un mode de réalisation, une fréquence de modulation globale comprise entre 0 et 4000 Hz, de préférence comprise entre 1 et 1000 Hz, de préférence comprise entre 1 et 100 Hz, de préférence d'environ 10 Hz est appliquée aux sources d'irradiation 14a, 14b, 14c des modules d'irradiation transcutanée 10 appliqués sur la tête 3 de l'utilisateur ; et une fréquence de modulation globale identique est appliquée aux sources d'irradiation 14a, 14b, 14c des modules d'irradiation transcutanée 10 appliqués sur l'abdomen 16 de l'utilisateur.

Il peut être alternativement prévu que la partie supérieure 1 du dispositif est soumise à une fréquence de modulation globale donnée (par exemple, 10 Hertz), et que la partie inférieure 9 du dispositif est soumise à une fréquence de modulation globale différente (par exemple 1000 Hertz).

On entend par « fréquence de modulation globale » l'établissement d'une alternance de plages non lumineuses et lumineuses. Dans les exemples de réalisation non limitatifs, les plages lumineuses sont de durée égale à la durée des plages non lumineuses. Cette fréquence de modulation globale est configurée par une console de pilotage électronique commandée par informatique, pour appliquer une fréquence d'émission contrôlée.

Avantageusement, chaque module d'irradiation transcutanée 10 comprend un aimant 18 qui s'étend selon un plan P et qui est positionné pour générer un champ magnétique statique, de préférence circulaire, compris entre 20 et 2 000 milliTeslas, de préférence compris entre 100 et 1000 milliTeslas, de préférence d'environ 200 milliTeslas. L'aimant 18 est de forme circulaire et est accolé sur la face interne du module d'irradiation transcutanée 10. Les rayonnements lumineux R générées par la source laser 14a, la diode électroluminescente (LED) 14b et la diode électroluminescente (LED) 14c s'étendent donc perpendiculairement au plan P de l'aimant 18 et à l'intérieur de cet aimant 18.

Concernant la source laser 14a, de préférence la diode laser 14a, celle-ci est ainsi soumise à une double pulsation :
- la première pulsation, intrinsèque, présente un train d'impulsion comprenant une fréquence de répétition comprise entre 1 et 25 kHz inclus, de préférence entre 10 et 15 kHz inclus, de préférence 15 kHz ou de préférence 10 kHz ;et
- la seconde pulsation, extrinsèque, et s'appliquant également aux LEDs 14b et 14c, comprenant une fréquence de répétition comprise entre 0 et 4000 Hz, de préférence comprise entre 1 et 1000 Hz, de préférence comprise entre 1 et 100 Hz, de préférence d'environ 10 Hz.

Les rayonnements électromagnétiques générés par les sources d'irradiation 14a, 14b, 14c traversent un guide optique 15 situé en partie inférieure du module d'irradiation transcutanée 10 à proximité des cheveux 16 et de la peau 17 de la tête 3 du patient (ou à proximité de l'abdomen 16). Le rayonnement pénètre alors vers la zone ciblée du cerveau ou de l'abdomen.

Le module d'irradiation transcutanée 10 peut être réalisé en une seule pièce, ou en deux pièces comme représenté sur la **Figure 3****.** Dans ce dernier cas, le module d'irradiation transcutanée 10 comprend une partie inférieure annulaire 10a, et une partie supérieure annulaire 10b. Les parties inférieure 10a et supérieure 10b peuvent s'assembler par tout moyen connu de l'homme du métier, notamment par un système à baïonnette, par vissage ou par encliquetage. Restant dans le cadre de l'invention, la structure du module peut être différente et adaptée par l'homme du métier.

Comme illustré sur la **Figure 3** pour ce mode de réalisation particulier, la nervure circulaire 11 est agencée sur la partie inférieure 10a qui comprend le guide optique 15, cette partie inférieure 10a pouvant ainsi rester en place sur l'anneau 2 entre deux traitements. La partie supérieure 10b comprend la carte électronique et la ou les sources d'irradiation, et peut venir être positionnée sur la partie inférieure au moment du traitement.

### Le support de modules

Pour assurer le positionnement fixe et précis des modules d'irradiation transcutanée 10 appliqués sur la tête 3 ou l'abdomen 16, il est prévu que le ou les modules soient agencés sur un support de modules comprenant un ou des anneaux réalisés dans un matériau souple et/ou élastique permettant d'assurer par emprise élastique le maintien fixe d'un module d'irradiation transcutanée. On entend par matériau souple et/ou élastique un matériau par exemple élastomère ou en caoutchouc qui autorise l'insertion d'un module de surface externe essentiellement cylindrique dans l'anneau par écartement dudit anneau et retour élastique contre le module. Le matériau pourra être souple sans être élastique, son écartement pour l'insertion et la fixation du module impliquant une sensible extension du matériau, le maintien du module dans l'anneau pouvant alors être assuré par friction. Le support comprend également des moyens de positionnement sur la zone à irradier. Ces moyens peuvent prendre la forme d'une sangle, mais peuvent également, ou en complément, tenir à la forme du support qui, en reposant sur la zone à traiter, s'adaptent à cette zone et sont maintenus en place du fait de cette forme de support adapté.

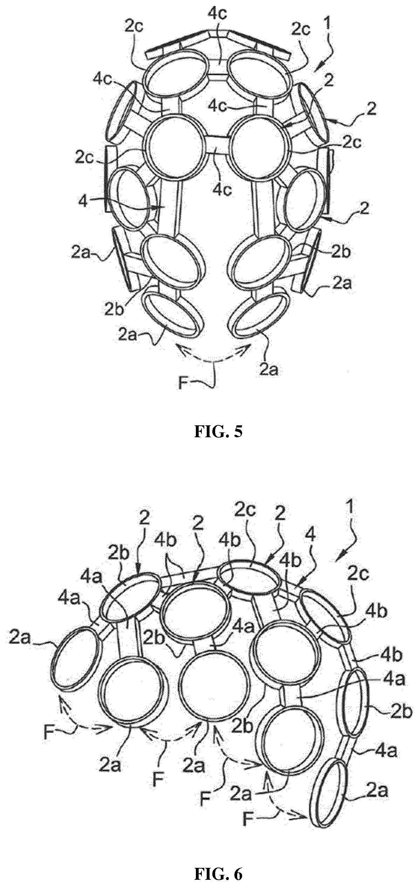

Le support de modules 20a destiné à la partie supérieure 1 du dispositif de l'invention doit s'adapter à la forme de la tête 3. On se réfère aux **Figures 5** à **10** pour décrire le support de modules de l'invention appliqué aux traitements neurologiques. Ce support de modules s'adapte dans sa structure à la réalisation du support de modules 20b de l'abdomen.

Le support 20a comprend une pluralité d'anneaux 2 de maintien de modules d'irradiation transcutanée 10 répartis à la surface de la tête 3 (pour des raisons de clarté, tous les anneaux ne sont pas référencés sur les figures). À titre d'exemple non limitatif, chaque anneau est réalisé en silicone, présente un rayon externe de 25 millimètres, un rayon interne de 23 millimètres (soit une épaisseur de 2 millimètres), et une hauteur de 6 millimètres. La hauteur doit être suffisante à la tenue d'un module qui sera décrit plus loin. En variante de l'utilisation de silicone, les anneaux peuvent être réalisés en caoutchouc, en matériau élastomère ou en tout autre matériau polymérique ou non qui est suffisamment souple pour permettent l'insertion et le maintien d'un module de surface externe essentiellement cylindrique.

Les anneaux 2 sont répartis symétriquement sur le support, de façon que lorsqu'ils sont positionnés sur la tête 3, l'axe de symétrie du support XX' coïncide avec l'axe médian XX' de la tête 3 de l'utilisateur. Les anneaux 2 sont reliés en entre eux par des éléments de jonction souples 4, réalisés par exemple dans le même matériau que les anneaux, dans cet exemple en silicone. Les éléments de jonction 4 sont précisément positionnés sur le support 20a pour permettre à la fois, au support 20a de s'adapter à la forme de la tête 3 de l'utilisateur, et pour permettre le maintien du support sur la tête 3, comme il sera décrit en détail plus loin.

Les anneaux 2 se répartissent de la façon suivante : le support 20a prévoit dix anneaux périphériques 2a, quatre anneaux de seconde périphérie 2b et quatre anneaux centraux 2c. Il est entendu que ce nombre précis et cette disposition précise des anneaux est donnée à titre d'exemple non limitatif. Le nombre d'anneaux 2 peut varier, leur position également, tout en restant dans le cadre de l'invention.

On se réfère à la **Figure 5** sur laquelle les anneaux 2a, 2b, 2c ont été sous numérotés 2a1, 2a2, 2a3, 2a4, 2a5, 2b1, 2b2, 2b3, 2b4, 2c1 et 2c2 pour décrire leur fonctionnalité, étant entendu que cette **Figure 5** ne représente qu'une moitié du support 20a, Les quatre anneaux périphériques 2a1, 2a2 et les deux anneaux de seconde périphérie 2b1 situés vers l'avant de la tête 3 sont destinés à atteindre le lobe frontal. Les six anneaux périphériques 2a2, 2a3 et 2a4 s'étendant depuis le second anneau périphérique avant sont destinés à atteindre le lobe temporal. Les quatre anneaux centraux 2c1, 2c2 et les quatre anneaux de seconde périphérie 2b2, 2b3 situés au-dessus des oreilles du patient sont destinés à atteindre le lobe pariétal. Les quatre anneaux périphériques 2a4, 2a5 les plus en arrière sont destinés à atteindre le cervelet. Et les deux anneaux de seconde périphérie 2b4 situés au-dessus de l'anneau périphérique le plus en arrière, et les deux anneaux périphériques 2a4 à l'avant des anneaux périphériques 2a5 les plus en arrière, sont destinés à atteindre le lobe occipital. Tous les anneaux 2 permettent également d'atteindre plus profondément le thalamus, l'hippocampe et les amygdales.

Les quatre anneaux centraux 2c sont reliés entre eux par des jonctions centrales 4a. Les anneaux de seconde périphérie 2b sont reliés aux anneaux centraux 2c par des jonctions de seconde périphérie 4b. Certains anneaux de seconde périphérie 2b sont également reliés entre eux par des jonctions de seconde périphérie 4b. Les anneaux périphériques 2a sont chacun relié à un anneau de second périphérie 2b par une jonction périphérique 4a. En revanche, les anneaux périphériques 2a ne sont pas reliés entre eux par des jonctions. Il est possible de prévoir que l'une des jonctions 4a, 4b, 4c comprend une zone plate permettant d'y solidariser une étiquette d'identification du patient.

L'absence de liaison entre les anneaux périphériques 2a permet au support 20a de s'adapter à la forme de la tête du patient en s'ouvrant plus ou moins. Restant dans le cadre de l'invention, certains anneaux périphériques 2a pourraient être reliés entre eux tout en conférant cette fonctionnalité d'adaptation. En revanche, si tous les anneaux périphériques 2a sont reliés entre eux, le support 20a ne pourra pas s'adapter à différentes formes de têtes.

Pour ce qui est des anneaux de seconde périphérie 2b, certains sont reliés entre eux pour que le support présente une tenue suffisante pour rester en place sur la tête et maintenir les modules en places dans les anneaux. Le fait que certains anneaux de seconde périphérie 2b ne soient pas reliés entre eux permet également d'entretenir la fonctionnalité d'adaptation du support 20a à toute forme de tête.

La présence ou non et la position des jonctions de seconde périphérie 4b et des jonctions périphériques 4a sont appréciées par l'homme du métier dans un compromis entre la rigidité du support 20a nécessaire pour lui permettre d'être maintenu en position sur la tête du patient et de maintenir le ou les modules, et le caractère d'adaptation du support 1 à toute forme de tête. La présence ou non de ces jonctions peut notamment varier selon le nombre d'anneaux 2 présents sur le support 1 ou encore la rigidité des matériaux employés pour réaliser les anneaux 2 et les jonctions 4.

En tout état de cause, il est essentiel qu'une partie au moins des anneaux périphériques 2a ne soient pas reliés entre eux, et de préférence, que tous les anneaux périphériques 2a ne soient pas reliés entre eux. Il apparaît également important pour la rigidité du support qu'au contraire, les anneaux centraux 2c soient reliés entre eux par des jonctions 4a. La liaison intégrale des anneaux centraux 2c dépend du nombre d'anneaux centraux 2c utilisés.

À titre d'exemple, le support de modules 20 pourra être réalisé en une seule pièce par moulage de silicone.

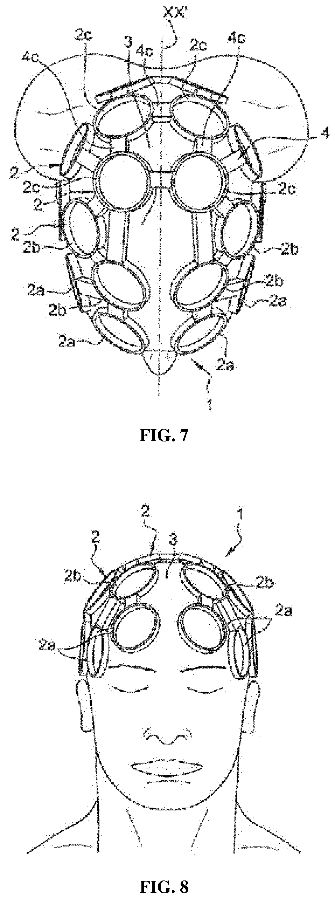

En référence à la **Figure 8****,** pour améliorer plus encore le maintien du support 20a sur la tête et pour positionner précisément les anneaux 2 vers les zones du cerveau à traiter, il est prévu une sangle à serrage contrôlé 5. Cette sangle 5 comprend une première partie de liaison 5a qui relie chaque anneau périphérique 2a en passant par un élément tubulaire 6 agencé au niveau du bord inférieur périphérique de chaque anneau périphérique 2a.

La sangle 5 comprend également une mentonnière 5b reliée à la première partie de liaison 5a par quatre éléments de liaison 5c, passant deux à deux de part et d'autre de l'oreille du patient.

On peut prévoir trois points de serrage 7a, 7b, 7c parmi lesquels deux points de serrage 7a, 7b sont situés à la jonction des éléments de liaison 5c et de la première partie de liaison 5a, et le troisième point de serrage est situé à la jonction entre la mentonnière 5b et les deux éléments de liaison 5c.

Comme précédemment décrit en référence à la **Figure 3****,** pour améliorer plus encore la tenue des modules d'irradiation transcutanée 10 dans les anneaux 2, on peut prévoir, la présence d'une rainure 8 au niveau de la face interne de l'anneau 2. Cette rainure 8 est destinée à coïncider avec la nervure circulaire 11 ménagée sur la surface externe du module associé 10. La présence de la rainure 8 permet non seulement d'améliorer la tenue du module, mais apporte également un positionnement plus précis du module pour que les rayonnements soient précisément dirigés vers les zones ciblées du cerveau ou de l'abdomen.

En référence à la **Figure 2****,** le support de modules 20b destiné à la partie inférieure 9 du dispositif de l'invention comprend également des anneaux 2 reliés entre eux par des jonctions 4, les anneaux 2 et les jonctions 4 étant identiques à ceux décrits précédemment pour le support de modules 20a. Le support de modules 20d comprennent à cet effet des anneaux périphériques 2a et des anneaux centraux 2c assurant la répartition des anneaux 2 selon quatre lignes aptes à s'étendre verticalement entre l'aine et l'estomac et horizontalement sur la largeur de l'abdomen 16. Comme pour le support de modules 20a, les anneaux périphériques 2a ne sont pas reliés entre eux par des jonctions 4 pour s'adapter au mieux à la morphologie abdominale de l'utilisateur.

Pour le maintien en position sur l'abdomen 16 de la partie inférieure 9 du dispositif de l'invention, il est possible de prévoir des boucles 19 solidarisées chacune aux extrémités opposées d'une même ligne d'anneaux 2 et dans lesquelles un bride formant ceinture peut être inséré en fixation.

### Système

La présente invention se rapporte également à un système comprenant le dispositif selon la présente invention, comprenant une console de pilotage comprenant une interface de commande pour configurer les paramètres de chaque des au moins une source d'irradiation 14a, 14b, 14c et une interface de communication pour délivrer des consignes numériques de pilotage au dit dispositif.

### Traitement

La présente invention se rapporte également au dispositif ou au système selon la présente invention, configuré pour la prévention et/ou le traitement de troubles neurologiques et/ou de maladies neurodégénératives chez un utilisateur en ayant besoin. En particulier, le dispositif ou le système selon la présente invention est configuré pour la prévention et/ou le traitement de la maladie d'Alzheimer, de la maladie de Parkinson et/ou de la maladie de Huntington.

La présente invention se rapporte également à une méthode de traitement des troubles neurologiques et/ou des maladies neurodégénératives, de préférence de la maladie d'Alzheimer, de la maladie de Parkinson et/ou de la maladie de Huntington, comprenant l'irradiation transcutanée d'un utilisateur en ayant besoin au moyen du dispositif ou du système selon la présente invention.

Dans un mode de réalisation, l'utilisateur est/a été diagnostiqué avec un trouble neurologique et/ou une maladie neurodégénérative, de préférence avec la maladie d'Alzheimer, la maladie de Parkinson et/ou la maladie de Huntington. L'utilisateur peut être diagnostiqué par un personnel médical (tel qu'un médecin ou une infirmière), un membre de sa famille ou une connaissance, qui reconnait, apprécie, détermine, confirme, conclue, pense ou décide que l'utilisateur est affecté avec un trouble neurologique et/ou une maladie neurodégénérative, de préférence avec la maladie d'Alzheimer, la maladie de Parkinson et/ou la maladie de Huntington.

Dans un mode de réalisation, l'utilisateur est un homme ou une femme, âgé de plus de 20 ans, plus de 30 ans, plus de 40 ans, plus de 50 ans, plus de 60 ans, plus de 70 ans ou plus de 80 ans.

Dans un mode de réalisation, le traitement d'irradiation transcutanée peut être réalisé par le dispositif ou le système de l'invention simultanément sur la tête ou sur l'abdomen, ou encore consécutivement. Dans un mode de réalisation, le temps d'irradiation transcutanée par cession est compris entre 5 et 60 minutes, de préférence entre 5 et 40 minutes, de préférence entre 10 et 30 minutes, de préférence est d'environ 25 minutes. Dans un mode de réalisation, le traitement d'irradiation transcutanée est réalisé une fois par jour, deux fois par jour ou trois fois par jour, de préférence une fois par jour. Dans un mode de réalisation, le traitement d'irradiation transcutanée est réalisé 1 à 7 fois par semaine, comme par exemple 1 jour par semaine, 2 jours par semaine, 3 jours par semaine, 4 jours par semaine, 5 jours par semaine, 6 jours par semaine ou 7 jours par semaine. Dans un mode de réalisation, la durée totale du traitement d'irradiation transcutanée est comprise entre 1 et 365 jours, selon la fréquence des traitements, la pathologie traitée et l'état général du patient.

Dans un mode de réalisation préférentiel, le traitement d'irradiation transcutanée est réalisé pendant une durée totale de 2 mois, à raison d'une séance de 25 minutes par jour pendant cinq jours tous les sept jours.

### EXEMPLES

### Exemple 1

Des tests ont été menés pour évaluer l'efficacité du dispositif de l'invention. Il a été plus précisément évalué l'efficacité du dispositif de l'invention sur l'atténuation de la pathologie induite par l'injection de bêta-amyloïde chez des souris. Ces tests ont également permis de déterminer le protocole d'irradiation permettant d'agir sur les maladies neurodégénératives telles que la maladie d'Alzheimer.

Le modèle animal utilisé pour tester le dispositif de l'invention est le modèle non-transgénique Aβ₂₅₋₃₅ de la maladie d'Alzheimer consistant en l'injection intracérébro-ventriculaire chez la souris du peptide amyloïde Aβ₂₅₋₃₅ sous forme oligomèrique. La présence de peptides amyloïdes a été identifiée dans le cerveau de malades Alzheimer ; le peptide Aβ₂₅₋₃₅ se trouve être l'un des plus neurotoxiques. Il a été montré que l'injection intracérébro-ventriculaire du peptide Aβ₂₅₋₃₅ résulte, sept jours après au niveau du cerveau, dans la présence d'une neuroinflammation et gliose réactive, l'activation de caspases pro-apoptotiques, un stress oxydant, une réduction du nombre de cellules pyramidales dans l'hippocampe, une perte de neurones cholinergiques et de sérieux troubles de mémoire. De manière très intéressante, l'injection du peptide Aβ₂₅₋₃₅ résulte dans l'installation d'une pathologie qui présente toutes les caractéristiques de la maladie d'Alzheimer chez l'homme avec en particulier l'accumulation d'espèces Aβ endogènes mais aussi une hyperphosphorylation de la protéine tau, comme observé dans la physiopathologie de la maladie d'Alzheimer.

Au jour 1, on a injecté à un groupe de souris « test » le peptide amyloïde Aβ₂₅₋₃₅ à raison de 9 nmol/souris afin de provoquer une toxicité amyloïde ; et à un autre groupe de souris « contrôle négatif » le peptide Sc.Aβ (scrambled amyloid-β protein 25-35) à raison également de 9 nmol/souris.

Une partie du groupe de souris « test » a été soumise à un traitement d'irradiation transcutanée du jour 1 (2 heures après l'injection du peptide amyloïde Aβ₂₅₋₃₅) au jour 10. Le traitement d'irradiation a été réalisé soit au niveau de la tête seule, soit au niveau de l'abdomen seul, soit au niveau de la tête et de l'abdomen. Les dispositifs d'irradiation utilisés sont soit selon l'invention, soit en dehors du champ de l'invention (notamment pour les dispositifs appliqués seulement sur la tête, ou seulement sur l'abdomen). Les traitements sont réalisés une ou deux fois par jour. Les différents dispositifs utilisés et comparés seront explicités plus loin.

Aux jours 8 à 10, des tests comportementaux sont réalisés sur tous les groupes de souris (Sc.Aβ sans traitement, Aβ₂₅₋₃₅ sans traitement, Aβ₂₅₋₃₅ avec traitement).

Le premier test comportemental réalisé le jour 8 évalue l'altération de la mémoire spatiale spontanée des souris au moyen d'un test d'évaluation de la performance d'alternance dans un labyrinthe en Y. Le labyrinthe comprend donc trois bras. Chaque souris est positionnée à l'extrémité d'un bras et peut se déplacer librement dans le labyrinthe pendant une session de 8 minutes. Le déplacement de chaque souris y compris les retours dans un même bras sont vérifiés visuellement. Une alternance est définie comme des entrées dans les trois bras à plusieurs occasions consécutives. Le nombre d'alternances maximales est le nombre total d'entrées dans les bras moins deux. Le pourcentage d'alternance est calculé comme étant : (le nombre d'alternances réelles/le nombre d'alternances maximales) x 100. Les résultats de ce premier test comportemental sont présentés sur les **Figures 11****,** **17** et **23****.**

Le second test comportemental réalisé les jours 9 et 10 évalue la mémoire contextuelle à long terme autrement dénommé « test d'évitement passif ». Ce test est réalisé le jour 10 en deux temps avec une séance de formation le jour 9. L'appareil faisant l'objet du test est une boîte à deux compartiments dont l'une est éclairée et l'autre plongée dans le noir et munie d'un plancher sous forme de grille. Une porte à fermeture de type guillotine sépare les deux compartiments. Des chocs peuvent être générés au niveau du planche en grille du compartiment obscur. Initialement, la porte séparant les deux compartiments est fermée. Pour la séance d'entraînement, chaque souris est placée dans le compartiment éclairé. Après 5 secondes, la porte est ouverte. Lorsque la souris pénètre dans le compartiment obscur, on génère des chocs électriques sur la grille. Au jour 10, la souris est de nouveau placée dans le compartiment éclairé, la porte fermée. La porte est ouverte et on mesure deux paramètres : le temps de latence, c'est-à-dire le temps au bout duquel la souris pénètre dans le compartiment obscur, et le temps d'échappement, c'est-à-dire le temps au bout duquel la souris sort du compartiment obscur. Les résultats de ces deux sous-tests (temps de latence et temps d'échappement) sont présentés sur les **Figures 12****,** **18** **et** **24** et sur les **Figures 13****,** **19** et **25****.**

Au jour 10, les souris sont euthanasiées. L'hippocampe et le cortex frontal des souris sont disséqués. On détermine les taux de peroxydation lipidique dans l'hippocampe en équivalents CHP par milligrammes de tissu et en pourcentage par rapport au groupe témoin (Sc.Aβ sans traitement). Les résultats sont présentés sur les **Figures 14****,** **20** et **26****.** On détermine également dans l'hippocampe par la méthode immuno-enzymatique ELISA le taux de GFAP (protéine acide fibrillaire gliale). Les résultats sont exprimés en pourcentage par rapport au groupe témoin (Sc.Aβ sans traitement) et présentés sur les **Figures 15****,** **21** et **27****.** On détermine en outre dans l'hippocampe par la méthode immuno-enzymatique ELISA le taux de TNFα (facteur de nécrose tumorale). Les résultats sont exprimés en pourcentage par rapport au groupe témoin (Sc.Aβ sans traitement) et présentés sur les **Figures 16****,** **22** et **28****.**

On détermine par ailleurs dans le cortex frontal les taux d'IL-1β (interleukine 1 beta) **(****Figure 23****)** et d'IL-6 (interleukine 6) **(****Figure 24****).** Ces deux cytokines rendent compte de l'état inflammatoire du tissu cérébral. On détermine également dans le cortex cérébral le taux des protéine Bax et Bcl2 dont le rapport traduit l'apoptose **(****Figure 25****),** de pTau Thr81 (protéine Tau) **(****Figure 26****)** et de Aβ₁₋₄₂ (protéine amyloide-β (1-42) **(****Figure 27****).** La protéine amyloïde-β (1-42) anormalement produite et la protéine Tau anormalement phosphorylée sont caractéristiques de la maladie d'Alzheimer.

On réalise enfin des analyses histologiques sur des coupes histologiques de la région C1 de l'hippocampe. Un comptage visuel est réalisé pour déterminer le nombre moyen d'astrocytes activés pour des séries de coupes en utilisant comme marqueur la protéine acide fibrillaire gliale (GFAP). Un comptage visuel est également réalisé pour déterminer le nombre moyen de cellules microgliales activées en utilisant comme marqueur la protéine Iba1. L'activation d'astrocytes et des cellules microgliales rendent compte des processus neuro-inflammatoires impliqués dans des pathologies neurologiques.

Il est à noter que pour les résultats présentés sur les **Figures 11** à **35**, les résultats obtenus par injection de Sc.Aβ sans traitement constituent un premier témoin de référence puisque cette injection n'a pas modifié le comportement des souris ni le taux de marqueurs testés. Les résultats obtenus par injection de Aβ₂₅₋₃₅ sans traitement constituent un second témoin de référence.

On notera également que les indications ###, ## et # signifient respectivement une totale, excellente et bonne adéquation avec le groupe témoin (Sc.Aβ sans traitement) et les indications ***,** et * signifient respectivement une absence totale, conséquente et sensible inadéquation avec le témoin (Sc.Aβ sans traitement).

On se réfère aux **Figures 11** à **16**. Ces figures illustrent les résultats obtenus par les tests précédemment évoqués pour des traitements d'irradiations réalisés une fois par jour sous les conditions opératoires suivantes :
- Référence 30 : injection de Sc.Aβ sans traitement (témoin 1).
- Référence 31 : injection de Aβ₂₅₋₃₅ sans traitement (témoin 2).
- Référence 32 : injection de Aβ₂₅₋₃₅ avec traitement de 10 minutes une fois par jour simultanément sur la tête et sur l'abdomen par le « dispositif A ».
- Référence 33 : injection de Aβ₂₅₋₃₅ avec traitement de 20 minutes une fois par jour simultanément sur la tête et sur l'abdomen par le « dispositif A ».
- Référence 34 : injection de Aβ₂₅₋₃₅ avec traitement de 5 minutes une fois par jour simultanément sur la tête et sur l'abdomen par le « dispositif B ».
- Référence 35 : injection de Aβ₂₅₋₃₅ avec traitement de 2,5 minutes une fois par jour simultanément sur la tête et sur l'abdomen par le « dispositif C ».
- Référence 36 : injection de Aβ₂₅₋₃₅avec traitement de 10 minutes une fois par jour simultanément sur la tête et sur l'abdomen par le « dispositif D ».
- Référence 37 : injection de Aβ₂₅₋₃₅avec traitement de 10 minutes une fois par jour simultanément sur la tête et sur l'abdomen par le « dispositif E ».
- Référence 38 : injection de Aβ₂₅₋₃₅ avec traitement de 10 minutes une fois par jour simultanément sur la tête et sur l'abdomen par le « dispositif F ».

« **Dispositif A »,** comprenant une partie supérieure 1 et une partie inférieure 9, comprenant chacune un module d'irradiation transcutanée 10 composé de :
- une diode électroluminescente (LED) générant un faisceau émettant dans le spectre infrarouge à une longueur d'onde de 850 nanomètres ;
- une diode électroluminescente (LED) générant un faisceau émettant dans le spectre rouge à une longueur d'onde de 625 nanomètres ;
- une diode laser de type pulsé générant un faisceau émettant dans le spectre infrarouge à une longueur d'onde de 850 nanomètres, présentant une durée d'impulsion comprise entre 120 et 150 nanosecondes, une fréquence de répétition du train d'impulsion de 10 kHz (soit 0,1 milliseconde), et une puissance impulsionnelle comprise entre 1 Watt et 7 Watt ;
- Une tension de 2,30 V ;
- un champ magnétique statique et circulaire de 200 milliTeslas.

Le traitement est établi en mode pulsé à une fréquence de modulation globale de 10 Hertz pour la tête et 1000 Hertz pour l'abdomen.

« **Dispositif B** », comprenant une partie supérieure 1 et une partie inférieure 9, comprenant chacune un module d'irradiation transcutanée 10 composé de :
- une diode électroluminescente (LED) générant un faisceau émettant dans le spectre infrarouge à une longueur d'onde de 850 nanomètres ;
- une diode électroluminescente (LED) générant un faisceau émettant dans le spectre rouge à une longueur d'onde de 625 nanomètres ;
- une diode laser de type pulsé générant un faisceau émettant dans le spectre infrarouge à une longueur d'onde de 850 nanomètres, présentant une durée d'impulsion comprise entre 120 et 150 nanosecondes, une fréquence de répétition du train d'impulsion de 10 kHz (soit 0,1 milliseconde) et une puissance impulsionnelle comprise entre 1 Watt et 7 Watt ;
- un champ magnétique statique et circulaire de 200 milliTeslas.

Le traitement est établi en mode continu, soit sans fréquence de modulation globale (0 Hertz) pour la tête et pour l'abdomen.

« **Dispositif C** », comprenant une partie supérieure 1 et une partie inférieure 9, comprenant chacune un module d'irradiation transcutanée 10 composé de :
- une diode électroluminescente (LED) générant un faisceau émettant dans le spectre rouge à une longueur d'onde de 625 nanomètres ;
- une diode laser de type continu générant un faisceau émettant dans le spectre infrarouge à une longueur d'onde de 850 nanomètres ; et
- un champ magnétique statique et circulaire de 200 milliTcslas.

Le traitement est établi en mode pulsé à une fréquence de modulation globale de 10 Hertz pour la tête et 1000 Hertz pour l'abdomen.

« **Dispositif D** », comprenant une partie supérieure 1 et une partie inférieure 9, comprenant chacune un module d'irradiation transcutanée 10 composé de :
- une diode électroluminescente (LED) générant un faisceau émettant dans le spectre infrarouge à une longueur d'onde de 850 nanomètres ;
- une diode électroluminescente (LED) générant un faisceau émettant dans le spectre rouge à une longueur d'onde de 625 nanomètres ;
- une diode laser de type pulsé générant un faisceau émettant dans le spectre infrarouge à une longueur d'onde de 850 nanomètres, présentant une durée d'impulsion comprise entre 120 et 150 nanosecondes, une fréquence de répétition du train d'impulsion de 10 kHz (soit 0,1 milliseconde) et une puissance impulsionnelle comprise entre 10 Watt et 13 Watt ; et
- un champ magnétique statique et circulaire de 200 milliTeslas.

Le traitement est établi en mode pulsé à une fréquence de modulation globale de 10 Hertz pour la tête et 1000 Hertz pour l'abdomen.

« **Dispositif E** », comprenant une partie supérieure 1 et une partie inférieure 9, comprenant chacune un module d'irradiation transcutanée 10 composé de :
- trois diodes électroluminescente (LED) générant un faisceau émettant dans le spectre infrarouge à une longueur d'onde de 850 nanomètre ; et
- un champ magnétique statique et circulaire de 200 milliTeslas.

Le traitement est établi en mode pulsé à une fréquence de modulation globale de 10 Hertz pour la tête et 1000 Hertz pour l'abdomen.

« **Dispositif F** », comprenant une partie supérieure 1 et une partie inférieure 9, comprenant chacune un module d'irradiation transcutanée 10 composé de :
- une diode électroluminescente (LED) générant un faisceau émettant dans le spectre infrarouge à une longueur d'onde de 850 nanomètres ;
- une diode électroluminescente (LED) générant un faisceau émettant dans le spectre rouge à une longueur d'onde de 625 nanomètres ;
- une diode laser de type pulsé générant un faisceau émettant dans le spectre infrarouge à une longueur d'onde de 850 nanomètres, présentant une durée d'impulsion comprise entre 80 et 100 nanosecondes, une fréquence de répétition du train d'impulsion de 10 kHz (soit 0,1 milliseconde) et une puissance impulsionnelle comprise entre 1 Watt et 7 Watt ;
- dépourvu de champ magnétique.

Le traitement est établi en mode pulsé à une fréquence de modulation globale de 10 Hertz pour la tête et 1000 Hertz pour l'abdomen.

On constate que le traitement le plus efficace sur les résultats présentés sur les **Figures 11** à **16** est le traitement dispensé par le « dispositif A » pour 10 minutes de traitement. À 20 minutes de traitement avec le « dispositif A », les résultats sont également très significatifs sauf pour le taux de GFAP et le taux de TNFα. Le traitement réalisé par le « dispositif F » (sans champ magnétique) présente d'excellent résultats pour le premier test comportemental **(****Figure 11****)** et sur les taux de GFAP et TNFα mais de moins bons résultats pour le second test comportemental et sur le taux de peroxydation lipidique. Le traitement dispensé par le « dispositif D » présente de bons, voire d'excellents, résultats pour le second test comportemental **(****Figures 12** **et** **13****)** ainsi que sur le taux de peroxydation lipidique et sur le taux de TNFα. Les « dispositifs A », « B », « D » et « F » présentent ainsi au moins en partie des résultats significatifs illustrant, par rapport au second témoin 31, une atténuation substantielle des pathologies tant comportementales qu'au niveau de marqueurs induite par le peptide Aβ₂₅₋₃₅. En revanche, les traitements dispensés par les « dispositifs C » et « E » ne montrent pas d'effet significatif. On constate la nécessité d'opérer dans le dispositif de l'invention avec un laser de type pulsé. Cette caractéristique importante présente un caractère surprenant à deux titres. En premier lieu, tous les dispositifs présentés sur les **Figures 11** à **16** sont en mode pulsé (10 Hertz pour la tête et 1000 Hertz pour l'abdomen). La nécessité d'opérer avec un laser de type également pulsé est donc étonnante puisqu'il en résulte une double pulsation (les fréquences de pulsation seront plus précisément évoquées en référence aux **Figures 17** à **22**). En outre, il pourrait être attendu que plus la puissance impulsionnelle est grande, plus l'effet est important, ce qui n'est pas le cas puisqu'avec un laser de puissance impulsionnelle comprise entre 10 et 13 Watts (« dispositif D »), les résultats sont moins bons que pour une puissance impulsionnelle comprise entre 1 et 7 Watt (« dispositif A » et « F » notamment).

On se réfère à présent aux **Figures 17** à **22**. Ces figures illustrent les résultats obtenus par les tests précédemment évoqués pour des traitements d'irradiations réalisés une ou deux fois par jour sous les conditions opératoires suivantes :
- Référence 30 : injection de Sc.Aβ sans traitement (témoin 1).
- Référence 31 : injection de Aβ₂₅₋₃₅ sans traitement (témoin 2).
- Référence 39 : injection de Aβ₂₅₋₃₅ avec traitement de 2,5 minutes deux fois par jour uniquement sur la tête par le « dispositif A » précédemment décrit.
- Référence 40 : avec traitement de 2,5 minutes deux fois par jour simultanément sur la tête et sur l'abdomen par le « dispositif A » précédemment décrit.
- Référence 41 : avec traitement de 5 minutes deux fois par jour simultanément sur la tête et sur l'abdomen par le « dispositif A » précédemment décrit.
- Référence 42 : avec traitement de 10 minutes une fois par jour simultanément sur la tête et sur l'abdomen par le « dispositif A » précédemment décrit (équivalent à la référence 32 des **Figures 11** à **16**).

On constate que le traitement le plus efficace sur les résultats présentés sur les **Figures 17** à **22** correspond à la référence 42, soit le traitement dispensé par le dispositif A pour 10 minutes de traitement une fois par jour simultanément sur la tête et sur l'abdomen. La référence 41, soit le même temps de traitement mais réparti sur deux cessions par jour, présente également une bonne efficacité mais sensiblement moindre que pour la référence 42. Les résultats correspondants aux références 39 et 40 sont en revanche insuffisant (à noter néanmoins de bons résultats obtenus pour la référence 40 sur le taux de TNFα), ce qui démontre d'une part la nécessité, pour l'atténuation de ce type de pathologie, d'un dispositif apte à être positionné sur la tête et sur l'abdomen pour réaliser un traitement d'irradiation sur ces deux zones, et d'autre part un temps minimum d'irradiation que l'on peut évaluer à 2,5 minutes environ.

On se réfère à présent aux **Figures 23** à **28**. Ces figures illustrent les résultats obtenus par les tests précédemment évoqués pour des traitements d'irradiations réalisés une fois par jour sous les conditions opératoires suivantes :
- Référence 30 : injection de Sc.Aβ sans traitement (témoin 1).
- Référence 31 : injection de Aβ₂₅₋₃₅ sans traitement (témoin 2).
- Référence 43 : injection de Aβ₂₅₋₃₅ avec traitement de 10 minutes une fois par jour simultanément sur la tête et sur l'abdomen par le « dispositif A » précédemment décrit (équivalent à la référence 32 des **Figures 11** à **16** et à la référence 42 des **Figures 17** à **22**).
- Référence 44 : injection avec traitement de 20 minutes une fois par jour uniquement sur la tête par le « dispositif A » précédemment décrit.
- Référence 45 : injection avec traitement de 20 minutes une fois par jour uniquement sur l'abdomen par le « dispositif A » précédemment décrit.
- Référence 46 : injection de Aβ₂₅₋₃₅ avec traitement de 10 minutes une fois par jour simultanément sur la tête et sur l'abdomen par le « dispositif A » précédemment décrit. Le traitement est établi en mode pulsé à 1000 Hertz pour la tête et pour l'abdomen.
- Référence 47 : injection de Aβ₂₅₋₃₅ avec traitement de 10 minutes une fois par jour simultanément sur la tête et sur l'abdomen par le « dispositif A » précédemment décrit. Le traitement est établi en mode pulsé à 10 Hertz pour la tête et pour l'abdomen.
- Référence 48 : injection de Aβ₂₅₋₃₅ avec traitement de 10 minutes une fois par jour simultanément sur la tête et sur l'abdomen par le « dispositif B » précédemment décrit. Le traitement est établi en mode continu (à 0 Hertz) pour la tête et pour l'abdomen.

On constate que les traitements correspondant aux références 43, 46 et 47 sont les plus efficaces, mettant en évidence que des traitements établis en mode pulsé à des fréquences comprises entre 10 et 1000 Hertz sont les plus efficaces. Le traitement correspondant à la référence 48 à 0 Hertz est également efficace en raison de la présence du laser utilisé de type pulsé. Les résultats correspondant aux références 44 et 45 confirment la caractéristique essentielle de la présente invention selon laquelle le dispositif de l'invention doit comprendre une partie positionnée sur la tête et une partie positionnée sur l'abdomen pour réaliser un traitement d'irradiation sur ces deux zones.

On se réfère à présent aux **Figures 29** à **33****.** Ces figures illustrent les résultats obtenus par les tests précédemment évoqués pour des traitements d'irradiations réalisés une fois par jour sous les conditions opératoires suivantes :
- Référence 30 : injection de Sc.Aβ sans traitement (témoin 1).
- Référence 31 : injection de Aβ₂₅₋₃₅ sans traitement (témoin 2).
- Référence 49 : avec traitement de 10 minutes une fois par jour simultanément sur la tête et sur l'abdomen par le « dispositif A » précédemment décrit. Le traitement est établi en mode pulsé à 10 Hertz pour la tête et pour l'abdomen.
- Référence 50 : avec traitement de 10 minutes une fois par jour simultanément sur la tête et sur l'abdomen par le « dispositif F » (sans champ magnétique) précédemment décrit. Le traitement est établi en mode pulsé à 10 Hertz pour la tête et 1000 Hertz pour l'abdomen.

On constate, comme pour les résultats correspondant aux références 32 et 38 des **Figures 11** à **16****,** que la présence du champ magnétique permet d'obtenir de meilleurs résultats notamment sur les marqueurs de l'inflammation cérébral **(****Figures 29** et **30**).

On se réfère enfin aux **Figures 34** et **35****.** Ces figures illustrent les résultats obtenus par les tests précédemment évoqués pour des traitements d'irradiations réalisés une fois par jour sous les conditions opératoires suivantes :
- Référence 30 : injection de Sc.Aβ sans traitement (témoin 1).
- Référence 31 : injection de Aβ₂₅₋₃₅ sans traitement (témoin 2).
- Référence 51 : avec traitement de 10 minutes une fois par jour simultanément sur la tête et sur l'abdomen par le « dispositif A » précédemment décrit. Le traitement est établi en mode pulsé à 10 Hertz pour la tête et pour l'abdomen.

On constate que l'injection de Aβ₂₅₋₃₅ a engendré l'activation des astrocytes (visuellement décelables par une extension de leurs ramifications par rapport à des astrocytes au repos) ainsi que l'activation des cellules microgliales (visuellement décelables par une morphologie amiboïde typique). Le traitement par le « dispositif A » de l'invention a fait significativement diminuer le nombre d'astrocytes et de cellules microgliales activées.

### Exemple 2

### Matériel et Méthodes

### Matériel

### Animaux

96 souris SWISS, âgées de 5 semaines et pesant entre 30 et 35 g, obtenues auprès de JANVIER (Saint Berthevin, France), ont été utilisées pour cette étude. Les souris ont été élevées en groupe avec un accès à l'eau et la nourriture *ad libitum* (#04C, Safe Diet, Augy France) à l'exception des périodes de test comportementaux. L'animalerie a été maintenue à une température et une humidité constante, sur un cycle jour/nuit de 12h/12h. L'identification des souris a été réalisée au moyen de marques réalisées sur la queue avec un feutre permanent.

La santé, l'aspect, l'activité t le poids des souris a été contrôlé quotidiennement au cours de l'étude.

### Dispositif de traitement utilisé

Le dispositif d'irradiation transcutanée « RGn530 » utilisé comprend une partie supérieure 1 et une partie inférieure 9, chaque partie comprenant un module d'irradiation transcutanée 10. Chaque module d'irradiation transcutanée 10 comprend :
- 1 diode laser 14a générant un faisceau émettant dans le spectre infrarouge, à une longueur d'onde de 850 nanomètres, train d'impulsion intrinsèque de 15 kHz, durée d'impulsion de 90 à 110 nanosecondes, puissance du pic d'impulsion de 1 à 11 Watts, tension d'alimentation de 4,24 Volts ;
- 1 diode électroluminescentes 14b générant un faisceau émettant dans le spectre rouge, à une longueur d'onde de 640 nanomètres ;
- 1 diode électroluminescente 14c générant un faisceau émettant dans le spectre infrarouge, à une longueur d'onde de 850 nanomètres ; et
- un champ magnétique statique et circulaire de 200 milliTeslas.

Le traitement est établi en mode pulsé à 10 Hertz pour la tête et pour l'abdomen.

La puissance impulsionnelle de la diode laser Pₘₐₓ telle que mentionnée dans cet exemple est comprise entre 11 et 13 Watts (correspondant à une durée comprise entre 130 et 150 nanosecondes).

La puissance impulsionnelle de la diode laser Pₘᵢₙ telle que mentionnée dans cet exemple est comprise entre 3 et 5 Watts (correspondant à une durée comprise entre 40 et 60 nanosecondes).

### Méthodes

### Préparation et injection des peptides β-amyloide

Les souris ont été, dans un premier temps, anesthésiées pendant 5 minutes avec de l'isoflurane 2,5%.

Après contention, les injections ont été réalisées dans le ventricule cérébral latéral au moyen d'une seringue en acier inoxydable de diamètre 28 et de 4 mm de longueur. 3 µL ont été injectés graduellement sur une durée de 30 secondes, l'aiguille a ensuite été retirée 30 secondes après injection (Haley et al., 1957. Br J Pharmacol Chemother. 12(1):12-5).

9 nmol du peptide β-amyloide (Aβ₂₅₋₃₅ - CAS 131602-53-4 Genepep, France France - véhicule utilisé : eau bidistillée stérile) ou du peptide contrôle « scramble » (Sc.AB - Genepep, France - véhicule utilisé : eau bidistillée stérile) ont été injectées dans chaque animal selon les protocoles précédemment décrits (Maurice et al., 1996. Brain Res. 731(1-2):249-53 ; Maurice et al., 1998. Neuroscience. 83(2):413-28 ; Meunier et al., 2006. Br J Pharmacol. 149(8):998-1012 ; Meunier et al., 2013. Eur J Pharmacol. 698(1-3):193-9 ; Villard et al., 2009. Neuropsychopharmacology. 34(6):1552-66 ; et Villard et al., 2011. J Psychopharmacol. 25(8):1101-17).

### Tests comportementaux

### Test Y-Maze pour l'évaluation de la mémoire spatiale à court terme

Au jour 8 de l'étude, toutes les souris ont été testées dans un test Y-Maze permettant l'évaluation de la mémoire spatiale à court terme.

Le test Y-Maze a été fabriqué en chlorure de polyvinyle gris selon Itoh et al. (1993. Eur J Pharmacol. 236(3):341-5) et Hiramatsu et al. (1999. Eur J Pharmacol. 367(2-3):151-5). Chaque bras a une longueur de 40 cm, une hauteur de 13 cm, une largeur au fond de 3 cm et une largeur aux bords de 10 cm. Les bras convergent à un angle identique.

Chaque souris est placée à l'extrémité d'un bras, et laissée libre de ses mouvements à l'intérieur du labyrinthe pendant 8 minutes. Les séries d'entrées dans un bras, incluant le retour dans un même bras, ont été observées. Une alternance (en anglais : *alternation*) est définie comme des entrées consécutives dans chacun des trois bras. Le nombre maximum d'alternances est calculé en soustrayant deux au nombre total d'entrées. Le pourcentage d'alternance correspond au nombre d'alternance divisé par le nombre d'alternance maximum multiplié par 100. Les paramètres considérés sont le pourcentage d'alternance, ou index de la mémoire (en anglais : *memory index*)*,* et le nombre total d'entrées, ou index d'exploration (en anglais : *exploration index*) (Maurice et al., 1996. Brain Res. 731(1-2):249-53 ; Maurice et al., 1998. Neuroscience. 83(2):413-28 ; Meunier et al., 2006. Br J Pharmacol. 149(8):998-1012 ; Meunier et al., 2013. Eur J Pharmacol. 698(1-3):193-9 ; Villard et al., 2009. Neuropsychopharmacology. 34(6):1552-66 ; et Villard et al., 2011. J Psychopharmacol. 25(8):1101-17).

Les animaux ayant un comportement extrême (*i.e.,* pourcentage d'alternance < 20 % ou > 90%) sont exclus des données. Dans le cadre de cette étude, aucun animal testé n'a été exclu des données.

### Test d'évitement passif pour l'évaluation de la mémoire contextuelle à long terme

Aux jours 9 et 10 de l'étude, toutes les souris ont été testées dans un test d'évitement passif (en anglais : *Step-Through Passive Avoidance - STPA*) permettant l'évaluation de la mémoire contextuelle à long terme.

L'appareil de test consiste en une boite (de dimension 15 x 20 x 15 cm de hauteur) comportant deux compartiments. Un des compartiments a des parois blanches et l'autre a des parois noires et un sol grillagé. Une porte guillotine sépare les compartiments. Une lampe de 60 Watts positionnée 40 cm au-dessus de l'appareil éclaire le compartiment blanc. Un choc électrique brouillé (0.3 mA durant 3 secondes) est envoyé grâce à un générateur de chocs électrique brouillés (Lafayette instruments, Lafayette, États-Unis).

La porte guillotine est maintenue fermée durant la phase d'entrainement. L'animal est placé dans le compartiment blanc durant cette phase. Après 5 secondes, la porte est ouverte. Lorsque la souris entre dans le compartiment noir et qu'elle a placé ses pattes sur le grillage, un choc électrique est envoyé dans le sol grillagé pendant 3 secondes. La latence d'entrée dans le compartiment noir (en anglais : *Step-Through Latency - STL*) et le nombre de vocalisation sont observés.

Le test de rétention est répété 24 heures après l'entrainement. Chaque souris est placée dans le compartiment blanc. Après 5 secondes, la porte guillotine est ouverte et les latences d'entrée et d'évitement sont observées jusqu'à 300 secondes (Meunier et al., 2006. Br J Pharmacol. 149(8):998-1012 ; Villard et al., 2009. Neuropsychopharmacology. 34(6):1552-66 ; et Villard et al., 2011. J Psychopharmacol. 25(8):1101-17).

Les animaux pour lesquels les latences observées sont inférieures à 10 secondes pendant l'entrainement et le test de rétention sont exclus des données. Dans le cadre de cette étude aucun animal testé n'a été exclu des données.

### Prélèvement des échantillons

Au jour 10 du protocole d'étude, les animaux ont été mis à mort par décapitation sans euthanasie.

Pour 12 animaux par groupe, le sang a été collecté dans des tubes EDTA et le plasma a été séparé.

Pour 12 animaux par groupe, le cerveau a été rapidement extrait et disséqué sur une plaque métallique refroidie par de la glace pour séparer deux hémihippocampes, le cortex et le reste du cerveau. Tous les tissus ont été congelés dans de la carboglace et stockés à -80°C.

Après laparotomie, le caecum a été incisé intégralement et son contenu aliquoté dans deux tubes Eppendorf.

### Mesure la peroxydation des lipides.

Six des douzes hippocampes prélevés au jour 10 pour chaque groupe ont été utilisés pour la mesure de l'activité de peroxydation des lipides telle que décrite dans Hermes-Lima et al. (1995. Free Radic Biol Med. 19(3) :271-80).

Après décongélation, les échantillons ont été homogénéisés dans du méthanol froid (1/10 masse/v), centrifugés à 1000 g pendant 5 minutes. Le surnageant a ensuite été prélevé dans un tube Eppendorf. Le volume de réaction pour chaque homogénat a été ajouté à une solution comprenant 1 mM FeSO₄, 0,25 mM H₂SO₄ et 1 mM xylénol orange, puis incubé pendant 30 minutes à température ambiante.

Après la lecture de l'absorbance à 580 nm (A₅₈₀1), 10 µL d'hydropéroxyde de cumène [1 mM] (CHP) a été ajouté à l'échantillon. L'absorbance à 580 nm a ensuite été mesurée après 30 minutes d'incubation à température ambiante (A₅₈₀2). Le niveau de péroxydation des lipides est calculé en équivalent-CHP selon la formule : (A₅₈₀1/A₅₈₀2 x [CHP nmol]) et exprimé par masse de tissus en pourcentage du niveau dans le groupe contrôle (Peptide contrôle (Sc.AB) - pas de traitement).

### Mesure du niveau de TNFα

Les hippocampes prélevés au jour 10 pour chaque groupe ont été utilisés pour la mesure de la quantité de TNFα par test ELISA (Référence EMTNFA, ThermoScientific, Etats-Unis).

Après décongélation, les échantillons ont été homogénéisés dans une solution tamponnée (150 mM Tris-NaCl, pH 7,5) et soniqués pendant 20 secondes. Après centrifugation (16500 g pendant 15 minutes à 4°C), le surnageant ou le plasma a ensuite été utilisé pour le test ELISA en accord avec les instructions du fabricant. Pour chaque test, l'absorbance a été mesurée à 450 nm et la concentration de TNFα calculée en utilisant la courbe standard.

Les résultats sont exprimés en pg par mg de tissu. Tous les échantillons ont été testé en duplicat.

### Analyse statistique

Toutes les valeurs, à l'exception de la latence d'évitement et de l'analyse de la composition du microbiote intestinal, sont exprimées en valeur moyenne +/- écart-type des moyennes.

L'analyse statistique utilisée pour chaque traitement est l'analyse de variance unilatérale (ANOVA, Valeur F) suivie d'un test de comparaison multiple de Dunnett. L'analyse des mesures de latence d'évitement ne suit pas une distribution gaussienne en raison de la valeur seuil maximale utilisée. Une analyse ANOVA non paramétrique Krustal-Wallis (Valeur H) a donc été utilisée. Les valeurs valeur p < 0.05 ont été considérées comme statistiquement significatives.

### Détermination de la composition du microbiote intestinal

Les populations bactériennes dans les échantillons de caecum prélevés au jour 10 du protocole ont été analysées par séquençage à haut débit (technologie MiSeq, Illumina, États-Unis) de la région variable V12-V4 de l'ARNr 16S amplifié par des amorces universelles.

Pour chaque échantillon, la librairie de séquence a été utilisée pour identifier les unités taxonomiques présentes. Pour les phyla *Firmicutes, Tenericutes* et *Deferribacteres,* une représentation des résultats sous forme d'histogramme a été utilisée et le test de Mann-Whitney a été utilisé pour l'analyse statistique.

### Protocole d'étude

Des groupes d'étude correspondant aux contrôles et à différents régimes de traitement d'irradiation transcutanée par le dispositif « RGn530 » ont été définis **(Tableau 1).**

Pour chacun des groupes, le peptide contrôle ou le peptide β-amyloïde a été injecté au jour 1 du protocole. Aux jours 1 à 9 du protocole, le traitement tel que défini dans le **Tableau 1** pour les différents groupes a été appliqué un fois par jour.

Au jour 8, la mémoire spatiale à court terme a été évaluée dans un test de type Y-maze. Aux jours 9 et 10 du protocole, la mémoire contextuelle à long terme a été évaluée dans un test d'évitement passif. Après le test au jour 10 du protocole, les animaux ont été sacrifiés et le prélèvement d'échantillons a été réalisé.

**Tableau 1**

| **Groupe de traitement** | **Taille de l'échantillon** | |
|---|---|---|
| 1 - Peptide contrôle (Sc.Aβ) - pas de traitement | 6 | 6 |
| 2 - Peptide β-amyloïde (Aβ₂₅₋₃₅) - pas de traitement | 6 | 6 |
| 3 - Peptide β-amyloïde (Aβ₂₅₋₃₅) - RGn530 tête et abdomen 3 minutes | 12 | - |
| 4 - Peptide β-amyloïde (Aβ₂₅₋₃₅) - RGn530 tête et abdomen 6 minutes | 12 | - |
| 5 - Peptide β-amyloïde (Aβ₂₅₋₃₅) - RGn530 tête et abdomen 6 minutes | 12 | - |
| 6 - Peptide β-amyloïde (Aβ₂₅₋₃₅) - RGn530X avec lentille tête et abdomen 6 minutes (pas de rasage) | - | 12 |
| 7 - Peptide β-amyloïde (Aβ₂₅₋₃₅) - RGn530Y Pmax tête et abdomen 4,5 minutes (rasage) | - | 12 |
| 8 - Peptide β-amyloïde (Aβ₂₅₋₃₅) - RGn530Z Pmin tête et abdomen 6 minutes (rasage) | - | 12 |
| **Total de souris** | 48 | 48 |

### Résultats

### Effet du traitement sur la mémoire spatiale à court terme (Y-Maze test)

L'injection de Aβ₂₅₋₃₅ conduit à une réduction significative de la mémoire spatiale à court terme par rapport aux souris injectées avec le peptide contrôle (Sc.Aβ).

Le traitement d'irradiation transcutanée à l'aide du dispositif « RGn530 », d'une durée de 3 minutes au niveau de la tête et l'abdomen, ne change pas de manière significative la réduction de la mémoire spatiale à court terme observée après l'injection de Aβ₂₅₋₃₅.

Le traitement d'irradiation transcutanée à l'aide du dispositif « RGn530 » d'une durée de 6 minutes (avec ou sans rasage) et 9 minutes au niveau de la tête et l'abdomen corrige de manière significative et complète la réduction de la mémoire spatiale à court terme observée après l'injection de Aβ₂₅₋₃₅.

Le traitement d'irradiation transcutanée à l'aide du dispositif « RGn530 » (avec Pₘₐₓ et Pₘᵢₙ) d'une durée de 4,5 minutes au niveau de la tête et l'abdomen corrige de manière significative et partielle la réduction de la mémoire spatiale à court terme observée après l'injection de Aβ₂₅₋₃₅.

Aucune réduction de la motricité des souris n'est observée.

Analyse statistique : One-way ANOVA
Alt% F_{(7;95)} 22,91, p > 0.001
Loc% F_{(7;95)} 0,9366, p > 0.05

Les résultats sont présentés **Figure 36****.**

### Effet du traitement sur la mémoire contextuelle à long terme (test d'évitement passif)

L'injection de Aβ₂₅₋₃₅ conduit à une réduction significative de la mémoire contextuelle à long terme par rapport aux souris injectées avec le peptide contrôle (Sc.Aβ).

Le traitement d'irradiation transcutanée à l'aide du dispositif « RGn530 » d'une durée de 3 minutes au niveau de la tête et l'abdomen ne change pas de manière significative la réduction de la mémoire contextuelle à long terme observée après l'injection de Aβ₂₅₋₃₅.

Le traitement d'irradiation transcutanée à l'aide du dispositif « RGn530 » d'une durée de 6 minutes (avec ou sans rasage) et 9 minutes au niveau de la tête et l'abdomen corrige de manière significative et complète la réduction de la mémoire contextuelle à long terme observée après l'injection de Aβ₂₅₋₃₅.

Le traitement d'irradiation transcutanée à l'aide du dispositif « RGn530 » (avec Pₘₐₓ et Pₘᵢₙ) d'une durée de 4,5 minutes au niveau de la tête et l'abdomen corrige de manière non significative et partielle la réduction mémoire contextuelle à long terme observée après l'injection de Aβ₂₅₋₃₅.

Aucune différence n'a été observée pour les profils de latence d'entrée (en anglais : *Step-Through Latency - STL*) et les profils de latence d'évitement (en anglais : *Escape Latency - EL).*

Les résultats sont présentés **Figure 37****.**

Analyse statistique : Krustkal-Wallis non-paramétrique ANOVA
STL H = 62.05, p > 0.0001
EL H = 57.03, p > 0.05

### Effet du traitement sur le stress oxydatif dans l'hippocampe (test de peroxydation des lipides)

L'injection de Aβ₂₅₋₃₅ conduit à une augmentation significative du niveau de stress oxydatif par rapport aux souris injectées avec le peptide contrôle (Sc.AB).

Le traitement d'irradiation transcutanée à l'aide du dispositif « RGn530 » d'une durée de 3 minutes au niveau de la tête et l'abdomen ne change pas de manière significative l'augmentation du niveau de stress oxydatif observée après l'injection de Aβ₂₅₋₃₅.

Le traitement d'irradiation transcutanée à l'aide du dispositif « RGn530 » d'une durée de 6 minutes (avec ou sans rasage) et 9 minutes au niveau de la tête et l'abdomen corrige de manière significative et complète l'augmentation du niveau de stress oxydatif observée après l'injection de Aβ₂₅₋₃₅.

Le traitement d'irradiation transcutanée à l'aide du dispositif « RGn530 » (avec Pₘₐₓ) d'une durée de 4,5 minutes au niveau de la tête et l'abdomen corrige de manière significative et complète l'augmentation du niveau de stress oxydatif observée après l'injection de Aβ₂₅₋₃₅.

Le traitement d'irradiation transcutanée à l'aide du dispositif « RGn530 » (avec Pₘᵢₙ) d'une durée de 4,5 minutes au niveau de la tête et l'abdomen corrige de manière significative et partielle l'augmentation du niveau de stress oxydatif observée après l'injection de Aβ₂₅₋₃₅.

Aucune différence n'a été observée pour les profils de latence de traversée (en anglais : *Step-Through Latency - STL*) et les profils de latence d'évitement (en anglais : *Escape Latency - EL*)*.*

Les résultats sont présentés **Figure 38****.**

Analyse statistique : One-way ANOVA
F_{(7;47)} 8,705, p < 0.001

### Effet du traitement sur l'inflammation dans l'hippocampe (niveau de TNFα)

L'injection de Aβ₂₅₋₃₅ conduit à une augmentation significative du niveau de TNFα par rapport aux souris injectées avec le peptide contrôle (Sc.Aβ).

Le traitement d'irradiation transcutanée à l'aide du dispositif « RGn530 » d'une durée de 3 minutes au niveau de la tête et l'abdomen corrige de manière significative et partielle l'augmentation du niveau de TNFα observée après l'injection de Aβ₂₅₋₃₅.

Le traitement d'irradiation transcutanée à l'aide du dispositif « RGn530 » d'une durée de 6 minutes (avec ou sans rasage) et 9 minutes au niveau de la tête et l'abdomen corrige de manière significative et complète l'augmentation du niveau de TNFα observée après l'injection de Aβ₂₅₋₃₅.

Le traitement d'irradiation transcutanée à l'aide du dispositif « RGn530 » (avec Pmin ou Pmax) d'une durée de 4,5 minutes au niveau de la tête et l'abdomen corrige de manière significative et complète l'augmentation du niveau de TNFα observée après l'injection de Aβ₂₅₋₃₅.

Les résultats sont présentés **Figure 39****.**

Analyse statistique : One-way ANOVA
F_{(7;47)} 220,9 p < 0.0001

### Effet du traitement sur la composition du microbiote intestinal (abondance des Firmicutes, Tenericutes et Deferribacteres)

L'injection de Aβ₂₅₋₃₅ conduit à une variation significative de l'abondance des *Firmicutes, Tenericutes* et *Deferribacteres* dans le ceacum, en comparaison avec les souris injectées avec le peptide contrôle (Sc.Aβ). En particulier, l'abondance des *Firmicutes* diminue significativement après injection de Aβ₂₅₋₃₅, tandis que celle des *Tenericutes* augmente significativement.

Le traitement d'irradiation transcutanée à l'aide du dispositif « RGn530 » d'une durée de 6 minutes (avec rasage) au niveau de la tête et l'abdomen entraîne une augmentation de l'abondance dans le ceacum des *Firmicutes* observée après l'injection de Aβ₂₅₋₃₅.

Par ailleurs, le traitement d'irradiation transcutanée à l'aide du dispositif « RGn530 » d'une durée de 6 minutes (avec rasage) au niveau de la tête et l'abdomen entraîne une diminution l'abondance des *Tenericutes* et des *Deferribacteres,* en comparaison avec les souris injectées avec le peptide Aβ₂₅₋₃₅.

Les résultats sont présentés **Figure 40****.**

### Conclusion

L'injection de Aβ₂₅₋₃₅ conduit à une réduction de la mémoire spatiale à court terme et de la mémoire contextuelle à long terme. L'injection de Aβ₂₅₋₃₅ conduit également à une augmentation du niveau de stress oxydatif et du niveau de TNFα.

Ces déficits comportementaux et biochimique sont atténués par un traitement quotidien d'irradiation transcutanée à l'aide du dispositif « RGn530 ». Ni le rasage, ni la variation de puissance (*i.e.,* Pₘᵢₙ *vs* Pₘₐₓ) n'affecte les résultats obtenus.

Le traitement quotidien d'irradiation transcutanée à l'aide du dispositif « RGn530 » entraîne une augmentation de l'abondance des *Firmicutes* dans le ceacum, atténuant ainsi la variation observée après l'injection de Aβ₂₅₋₃₅ ; tandis que le même traitement quotidien d'irradiation transcutanée provoque une diminution de l'abondance des *Tenericutes* et des *Deferribacteres,* en comparaison avec l'abondance observée chez les souris injectées avec le peptide Aβ₂₅₋₃₅ non-traitées.

De manière particulièrement intéressante, la variation de l'abondance des *Firmicutes,* des *Tenericutes* et des *Deferribacteres* observée dans le modèle de maladie d'Alzheimer utilisé dans cette étude, se retrouve, associée à deux autres maladies neurodégénératives : la maladie de Huntington et la maladie de parkinson (Tremlett et al., 2017. Ann Neurol. 81(3):369-382). Cette constatation et les observations de cette étude sur l'effet du traitement sur la composition du microbiote intestinal suggèrent donc une utilité du dispositif selon l'invention dans le traitement de la maladie Huntington et de la maladie de Parkinson. En accord avec cette analyse, un traitement antibiotique, conduisant à une réduction de l'abondance des *Firmicutes* manière similaire à la maladie d'Alzheimer, a été associé à une réduction de la motricité, symptôme dans la maladie de Parkinson (Parashar et al., 2017. Parkinsonism Relat Disord. 38:1-7)*.*

## Revendications

1. Un dispositif d'irradiation transcutanée, comprenant :
- une partie supérieure (1) apte à être positionnée sur la tête (3) d'un utilisateur et comprenant au moins un module d'irradiation transcutanée (10) composé d'au moins une source d'irradiation (14a, 14b, 14c), et
- une partie inférieure (9) apte à être positionnée sur l'abdomen (16) de l'utilisateur et comprenant au moins un module d'irradiation transcutanée (10) composé d'au moins une source d'irradiation (14a, 14b, 14c),
chaque module d'irradiation transcutanée (10) comprenant au moins une source laser de type pulsé (14a),
le dispositif étant **caractérisé en ce qu'**une fréquence de modulation globale est appliquée aux sources d'irradiation (14a, 14b, 14c), la source laser de type pulsé (14a) étant en conséquence soumise à une double pulsation : une première pulsation, intrinsèque et une seconde pulsation, extrinsèque et résultant de l'application de la fréquence de modulation globale à la première pulsation.

2. Le dispositif selon la revendication **1,** dans lequel la fréquence de modulation globale est comprise entre 1 et 1000 Hz, de préférence entre 1 et 100 Hz, de préférence d'environ 10 Hz.

3. Le dispositif selon la revendication **1** ou la revendication **2,** dans lequel la source laser de type pulsé (14a) génère un faisceau émettant dans le spectre infrarouge, ledit faisceau présentant une longueur d'onde comprise entre 700 et 1200 nanomètres et un train d'impulsion comprenant :
- une durée d'impulsion comprise entre 20 et 200 nanosecondes,
- une fréquence de répétition du train d'impulsion comprise entre 1 et 25 kHz inclus, de préférence entre 10 et 15 kHz inclus, de préférence est d'environ 15 kHz ;
- une puissance impulsionnelle comprise entre 0,5 et 12 Watts inclus ; et
- une tension comprise entre 2 et 5 Volts inclus.

4. Le dispositif selon l'une quelconque des revendications **1** à **3,** dans lequel la double pulsation à laquelle la source laser de type pulsé (14a) est soumise comprend :
- la première pulsation, intrinsèque, présentant une fréquence de répétition du train d'impulsion comprise entre 1 et 25 kHz, de préférence entre 10 et 15 kHz, et
- la seconde pulsation, extrinsèque et résultant de l'application de la fréquence de modulation globale, comprenant une fréquence de répétition comprise entre 1 et 1000 Hz, de préférence comprise entre 1 et 100 Hz, de préférence d'environ 10 Hz.

5. Le dispositif selon l'une quelconque des revendications **1** à **5, caractérisé en ce que** chaque module d'irradiation transcutanée (10) comprend en outre au moins une diode électroluminescente (14b, 14c) générant un faisceau émettant dans le spectre visible ou dans le spectre infrarouge.

6. Le dispositif selon l'une quelconque des revendications **1** à **5, caractérisé en ce que** chaque module d'irradiation transcutanée (10) comprend au moins :
- une source laser de type pulsé (14a) générant un faisceau émettant dans le spectre infrarouge ;
- une diode électroluminescente ou une source laser générant un faisceau émettant dans le spectre rouge (14b), et ;
- une diode électroluminescente générant un faisceau émettant dans le spectre infrarouge (14c).

7. Le dispositif selon l'une quelconque des revendications **1** à **6, caractérisé en ce que** chaque module d'irradiation transcutanée (10) comprend en outre une source générant un champ magnétique statique (18), de préférence un aimant ou un électro-aimant.

8. Le dispositif selon la revendication **7, caractérisé en ce que** la source générant le champ magnétique statique (18) comprend une forme d'anneau destiné à être agencé perpendiculairement au plan duquel est générée l'irradiation transcutanée par la au moins une source d'irradiation (14a, 14b, 14c).

9. Le dispositif selon l'une quelconque des revendications **1** à **8, caractérisé en ce qu'**il comprend au moins deux modules d'irradiation transcutanée (10) disposés sur la tête de l'utilisateur, de préférence disposés symétriquement ; et au moins deux modules d'irradiation transcutanée (10) disposés sur l'abdomen de l'utilisateur, de préférence disposés symétriquement.

10. Le dispositif selon l'une quelconque des revendications **1** à **9, caractérisé en ce qu'**au moins une source d'irradiation (14a, 14b, 14c) est maintenue au sein d'un moyen de maintien dont la forme est sensiblement cylindrique, ledit moyen de maintien étant destiné à être au contact d'un patient de sorte que le faisceau soit émis parallèlement à l'axe du cylindre.

11. Le dispositif selon l'une des revendications **1 à 10, caractérisé en ce qu'**il comprend des moyens d'application de l'irradiation par la au moins une source d'irradiation (14a, 14b, 14c), et **en ce que** les moyens d'applications de l'irradiation comprennent un module de synchronisation des émissions du module d'irradiation transcutanée de la partie supérieure (1) avec les émissions du module d'irradiation transcutanée de la partie inférieure (9), lesdits faisceaux étant émis à une fréquence de modulation globale d'environ 9 à 11 Hz, de préférence d'environ 10 Hz pour chacun des modules d'irradiation transcutanée (10) de la partie supérieure (1) et de la partie inférieure (9).

12. Un système comprenant le dispositif selon l'une quelconque des revendications **1** à **11, caractérisé en ce qu'**il comprend une console de pilotage comprenant une interface de commande pour configurer les paramètres de chacune des au moins une source d'irradiation (14a, 14b, 14c) et une interface de communication pour délivrer des consignes numériques de pilotage au dit dispositif.

13. Le dispositif selon l'une quelconque des revendications **1** à **11,** ou le système selon la revendication **12,** configuré pour la prévention ou le traitement des troubles neurologiques et/ou des maladies neurodégénératives, de préférence pour la prévention ou le traitement de la maladie d'Alzheimer, de la maladie de Parkinson et/ou de la maladie de Huntington.

## Patentansprüche

1. Vorrichtung zur transkutanen Bestrahlung, umfassend
- einen oberen Teil (1), der imstande ist, auf dem Kopf (3) eines Benutzers positioniert zu sein und mindestens ein Modul zur transkutanen Bestrahlung (10) umfasst, das aus mindestens einer Bestrahlungsquelle (14a, 14b, 14c) besteht, und
- einen unteren Teil (9), der imstande ist, auf dem Bauch (16) des Benutzers positioniert zu sein und mindestens ein Modul zur transkutanen Bestrahlung (10) umfasst, das aus mindestens einer Bestrahlungsquelle (14a, 14b, 14c) besteht,
wobei jedes Modul zur transkutanen Bestrahlung (10) mindestens eine Laserquelle vom gepulsten Typ (14a) umfasst,
wobei die Vorrichtung **dadurch gekennzeichnet ist, dass** eine globale Modulationsfrequenz auf die Bestrahlungsquellen (14a, 14b, 14c) angewendet wird, wobei die Laserquelle vom gepulsten Typ (14a) folglich einer doppelten Pulsation ausgesetzt ist: einer ersten intrinsischen Pulsation und einer zweiten extrinsischen Pulsation und im Ergebnis der Anwendung der globalen Modulationsfrequenz auf die erste Pulsation.

2. Vorrichtung nach Anspruch 1, wobei die globale Modulationsfrequenz zwischen 1 und 1000 Hz, vorzugsweise zwischen 1 und 100 Hz, vorzugsweise bei etwa 10 Hz, liegt.

3. Vorrichtung nach Anspruch 1 oder Anspruch 2, wobei die Laserquelle vom gepulsten Typ (14a) einen im Infrarotspektrum emittierenden Strahl erzeugt, wobei der Strahl eine Wellenlänge zwischen 700 und 1200 Nanometern und eine Impulsfolge aufweist, umfassend:
- eine Impulsdauer, die zwischen 20 und 200 Nanosekunden liegt,
- eine Wiederholungsfrequenz der Impulsfolge, die zwischen 1 und 25 kHz einschließlich, vorzugsweise zwischen 10 und 15 kHz einschließlich, vorzugsweise bei etwa 15 kHz liegt;
- eine Impulsleistung, die zwischen 0,5 und 12 Watt einschließlich liegt; und
- eine Spannung, die zwischen 2 und 5 Volt einschließlich liegt.

4. Vorrichtung nach einem der Ansprüche 1 bis 3, wobei die doppelte Pulsation, der die Laserquelle vom gepulsten Typ (14a) ausgesetzt ist, umfasst:
- die erste intrinsische Pulsation, die eine Wiederholungsfrequenz der Impulsfolge aufweist, die zwischen 1 und 25 kHz, vorzugsweise zwischen 10 und 15 kHz, liegt, und
- die zweite extrinsische Pulsation als Ergebnis der Anwendung der globalen Modulationsfrequenz, umfassend eine Wiederholungsfrequenz zwischen 1 und 1000 Hz, vorzugsweise zwischen 1 und 100 Hz, vorzugsweise von etwa 10 Hz.

5. Vorrichtung nach einem der Ansprüche 1 bis 5, **dadurch gekennzeichnet, dass** jedes Modul zur transkutanen Bestrahlung (10) ferner mindestens eine Leuchtdiode (14b, 14c) umfasst, die einen Strahl erzeugt, der im sichtbaren Spektrum oder im Infrarotspektrum emittiert.

6. Vorrichtung nach einem der Ansprüche 1 bis 5, **dadurch gekennzeichnet, dass** jedes Modul zur transkutanen Bestrahlung (10) mindestens umfasst:
- eine Laserquelle (14a) vom gepulsten Typ, die einen im Infrarotspektrum emittierenden Strahl erzeugt;
- eine Leuchtdiode oder eine Laserquelle, die einen im roten Spektrum (14b) emittierenden Strahl erzeugt, und
- eine Leuchtdiode, die einen im Infrarotspektrum (14c) emittierenden Strahl erzeugt.

7. Vorrichtung nach einem der Ansprüche 1 bis 6, **dadurch gekennzeichnet, dass** jedes Modul zur transkutanen Bestrahlung (10) ferner eine Quelle zur Erzeugung eines statischen Magnetfelds (18), vorzugsweise einen Magneten oder Elektromagneten, umfasst.

8. Vorrichtung nach Anspruch 7, **dadurch gekennzeichnet, dass** die das statische Magnetfeld erzeugende Quelle (18) eine Ringform umfasst, die dazu bestimmt ist, senkrecht zu der Ebene eingerichtet zu sein, von der die transkutane Bestrahlung durch die mindestens eine Bestrahlungsquelle (14a, 14b, 14c) erzeugt wird.

9. Vorrichtung nach einem der Ansprüche 1 bis 8, **dadurch gekennzeichnet, dass** sie mindestens zwei Module zur transkutanen Bestrahlung (10) umfasst, die auf dem Kopf des Benutzers vorzugsweise symmetrisch angeordnet sind; und mindestens zwei Module zur transkutanen Bestrahlung (10), die auf dem Bauch des Benutzers vorzugsweise symmetrisch angeordnet sind.

10. Vorrichtung nach einem der Ansprüche 1 bis 9, **dadurch gekennzeichnet, dass** mindestens eine Bestrahlungsquelle (14a, 14b, 14c) in einem Haltemittel gehalten wird, dessen Form im Wesentlichen zylindrisch ist, wobei das Haltemittel dazu bestimmt ist, derart in Kontakt mit einem Patienten zu sein, dass der Strahl parallel zur Achse des Zylinders emittiert wird.

11. Vorrichtung nach einem der Ansprüche 1 bis 10, **dadurch gekennzeichnet, dass** sie Mittel zur Anwendung der Bestrahlung durch die mindestens eine Bestrahlungsquelle (14a, 14b, 14c) umfasst, und dass die Mittel zur Anwendung der Bestrahlung ein Synchronisationsmodul der Emissionen des Moduls zur transkutanen Bestrahlung des oberen Teils (1) mit den Emissionen des Moduls zur transkutanen Bestrahlung des unteren Teils (9) umfassen, wobei die Strahlen in einer globalen Modulationsfrequenz von etwa 9 bis 11 Hz, vorzugsweise von etwa 10 Hz für jedes der Module zur transkutanen Bestrahlung (10) des oberen Teils (1) und des unteren Teils (9) emittiert werden.

12. System, umfassend die Vorrichtung nach einem der Ansprüche 1 bis 11, **dadurch gekennzeichnet, dass** es eine Steuerkonsole umfasst, die eine Steuerschnittstelle zum Konfigurieren der Parameter jeder der mindestens einen Bestrahlungsquelle (14a, 14b, 14c) und eine Kommunikationsschnittstelle zum Übertragen der digitalen Steueranweisungen an die Vorrichtung umfasst.

13. Vorrichtung nach einem der Ansprüche 1 bis 11 oder System nach Anspruch 12, die zur Prävention oder Behandlung von neurologischen Störungen und/oder neurodegenerativen Erkrankungen, vorzugsweise zur Prävention oder Behandlung der Alzheimer-Krankheit, der Parkinson-Krankheit und/oder der Huntington-Krankheit, ausgelegt ist.

## Claims

1. A transcutaneous irradiation device, comprising :
- an upper part (1) adapted to be positioned on the head (3) of a user and comprising at least one transcutaneous irradiation module (10) composed at least one irradiation source (14a, 14b, 14c), and
- a lower part (9) adapted to be positioned on the user's abdomen (16) and comprising at least one transcutaneous irradiation module (10) consisting at least one irradiation source (14a, 14b, 14c),
each transcutaneous irradiation module (10) comprising at least one pulsed-type laser source (14a),
the device being **characterized in that** an overall modulation frequency is applied to the irradiation sources (14a, 14b, 14c), the pulsed-type laser source (14a) consequently being subjected to a double pulsation: one first, intrinsic pulsation and a second, extrinsic pulsation resulting from the application of the global modulation frequency to the first pulsation.

2. The device according to claim 1, wherein the overall modulation frequency is between 1 and 1000 Hz, preferably between 1 and 100 Hz, preferably around 10 Hz.

3. The device according to claim 1 or claim 2, wherein the pulsed-type laser source (14a) generates a beam emitting in the infrared spectrum, said beam having a wavelength between 700 and 1200 nanometres and pulse train comprising :
- a pulse duration of between 20 and 200 nanoseconds,
- a pulse train repetition frequency of between 1 and 25 kHz inclusive, preferably between 10 and 15 kHz inclusive, more preferably about 15 kHz;
- a pulse power of between 0.5 and 12 Watts inclusive; and
- voltage between 2 and 5 volts inclusive.

4. The device according any one of claims 1 to 3, wherein the double pulse to which the pulsed type laser source (14a) is subjected comprises
- the first, intrinsic pulsation, with a frequency of pulse train repetition between 1 and 25 kHz preferably between 10 and 15 kHz, and
- the second, extrinsic pulsation resulting application of the global modulation frequency, comprising a frequency of Repetition between 1 and 1000 Hz, preferably between 1 and 100 Hz, preferably about 10 Hz.

5. The device according to any one of claims 1 to 5, **characterized in that** each transcutaneous irradiation module (10) comprises at least one light-emitting diode (14b, 14c) generating a beam emitting in the visible spectrum or in the infrared spectrum.

6. The device according to any one of claims 1 to 5, **characterized in that** each transcutaneous irradiation module (10) comprises at least :
- a pulsed laser source (14a) generating a beam emitting in the infrared spectrum ;
- a light-emitting diode or laser source generating a beam emitting in the red spectrum (14b), and;
- a light-emitting diode generating a beam emitting in the infrared spectrum (14c).

7. The device according to any one of claims 1 to 6, **characterized in that** each transcutaneous irradiation module (10) further comprises a source generating a static magnetic field (18), preferably a magnet or an electromagnet.

8. The device according to claim 7, **characterized in that** the source generating the static magnetic field (18) comprises a ring shape intended to be perpendicular to the plane from which the transcutaneous irradiation is generated by the at least one irradiation source (14a, 14b, 14c).

9. The device according to any one of claims 1 to 8, characterized that it comprises at least two transcutaneous irradiation modules (10) arranged on the user's head, preferably arranged symmetrically; and at least two transcutaneous irradiation modules (10) arranged on the user's head. the user's abdomen, preferably arranged symmetrically.

10. The device according to any one of claims 1 to 9, **characterized in** at least one irradiation source (14a, 14b, 14c) is held within a holding means whose shape is substantially cylindrical, said holding means being intended to be in contact with a patient in such a way that the beam is emitted parallel to the axis of the cylinder.

11. The device according to one of claims 1 to 10, **characterized in that** it means for applying irradiation by the at least one irradiation source (14a, 14b, 14c), and **in that** the irradiation application means comprise a module for synchronising the emissions transcutaneous irradiation module of the upper part (1) with the emissions of the transcutaneous irradiation module of the lower part (9), said beams being emitted at an overall modulation frequency of approximately 9 to 11 Hz, preferably about 10 Hz for each of the transcutaneous irradiation modules (10) in the upper part (1) and the lower part(9).

12. A system comprising the device according to any one of claims 1 to 11, characterized that it comprises a control console comprising a control interface for configuring the parameters of each of the at least one irradiation source (14a, 14b, 14c) and a communication interface for delivering digital control instructions to said device.

13. The device according to any one of claims 1 to 11, or the system according to claim 12, configured for the prevention or treatment of neurological disorders and/or neurodegenerative diseases, preferably for the prevention or treatment Alzheimer's disease, Parkinson's disease and/or Huntington's disease.
